# EUROPÄISCHE PATENTSCHRIFT

(11) **EP 0 475 273 B1**
(45) Veröffentlichungstag und Bekanntmachung des Hinweises auf die Patenterteilung: **07.04.1999**
(21) Anmeldenummer: 91114986.2
(22) Anmeldetag: 05.09.1991
(51) Int. Cl.: C09K 19/34, C09K 19/30, C07C 25/24, C07D 239/26, C07D 213/26, G02F 1/1337

(54) **Flüssigkristallines Gemisch mit halogenierten Alkenyl-Verbindungen**
Liquid crystal mixture containing halogenated alcenyl compounds
Mélange de cristaux liquides contenant des composés alcényl halogénés

(30) Priorität: 14.09.1990 CH 2992/90; 14.05.1991 CH 1434/91
(43) Veröffentlichungstag der Anmeldung: 18.03.1992
(73) Patentinhaber: MERCK PATENT GmbH, 64293 Darmstadt (DE)
(72) Erfinder: Buchecker, Richard, CH-8008 Zürich (CH); Germann, Alfred, CH-4058 Basel (CH); Schadt, Martin, CH-4411 Seltisberg (CH); Vilinger, Alois, CH-4055 Basel (CH)

(56) Entgegenhaltungen:
- EP-A- 0 168 683
- EP-A- 0 315 014
- WO-A-91/02709

## Beschreibung

Die vorliegende Erfindung betrifft neue flüssigkristalline Gemische, die halogenierte Dreiring-Alkenyl-Verbindungen enthalten, sowie deren Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Verbindungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("supertwisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben. Sie sollen bei üblichen Betriebstemperaturen eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen.

So werden beispielsweise in der EP-A-0 315 014 halogenierte Flüssigkristall-Verbindungen mit 3E-Alkenyl-, 4-Alkenyl-, 2E-Alkenyloxy- oder 3-Alkenyloxy-Seitenketten offenbart. Aus der WO 91/02709, einem Stand der Technik nach A 54(3), sind Phenylcyclohexane mit Alkenylseitenketten bekannt. Flüssigkristalline Mischungen mit halogenierten Vinyl- oder 1-E-Alkenyl-Verbindungen werden dort nicht genannt. Neben dem generellen Interesse an Verbindungen mit hoher optischer Anisotropie besteht in letzter Zeit ein vermehrtes Interesse an Materialien mit niedriger optischer Anisotropie, insbesondere für aktiv adressierte Flüssigkristallanzeigen, z.B. für TFT-Anwendungen ("thin film transistor" = Dünnfilmtransistor) in Fernsehgeräten, wobei jedoch Effekte, wie das Auftreten von hochgeordneten smektischen Phasen oder eine Erhöhung der Schwellenspannung und der Ansprechzeiten, die bei solchen Materialien häufig beobachtet werden, möglichst vermieden werden sollten.

Da Flüssigkristalle in der Regel als Mischungen mehrerer Komponenten zur Anwendung gelangen, ist es wichtig, dass die Komponenten untereinander gut mischbar sind.

Gegenstand der Erfindung ist ein flüssigkristallines Gemisch mit mindestens 2 Komponenten, dadurch gekennzeichnet, daß mindestens eine Komponente eine Verbindung Formel I 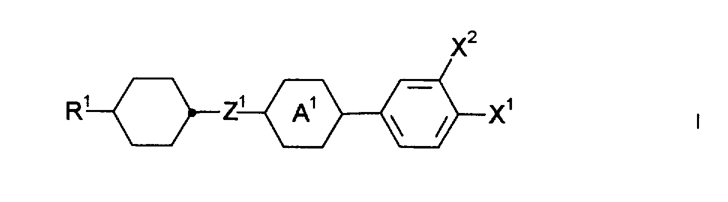 ist,
worin
- Z¹: eine einfache Kovalenzbindung oder -CH₂CH₂- bezeichnet;
- Ring A¹: 1,4-Phenylen, Pyrimidin-2,5-diyl, Pyridin-2,5-diyl oder, wenn Z¹ für -CH₂CH₂- steht, auch trans-1,4-Cyclohexylen darstellt;
- X¹: für Fluor oder Chlor steht;
- X²: Fluor oder Chlor bedeutet,
- R¹: Vinyl oder 1E-Alkenyl mit 3 bis 12 Kohlenstoffatomen bedeutet,
wobei der Anteil an Verbindungen der Formel I 5-30 Gew.-% beträgt.

Die erfindungsgemässen Verbindungen sind Flüssigkristalle mit breiter nematischer Phase und vergleichsweise hohem Klärpunkt. Sie haben eine überraschend hohe dielektrische Anisotropie bei relativ niedriger Rotationsviskosität und führen zu niedriger Schwellenspannung und kurzen Ansprechzeiten.

Die erfindungsgemässen Verbindungen eignen sich für Anzeigevorrichtungen mit verdrillt nematischer Struktur und TFT-Zellen. Sie können dank ihrer guten Mischbarkeit untereinander und mit bekannten Flüssigkristallmaterialien in vergleichsweise hoher Konzentration verwendet werden. Sie eignen sich insbesondere als Komponenten nematischer und cholesterischer Gemische.

Der obige Ausdruck "lE-Alkenyl" umfasst geradkettige oder verzweigte Reste mit 3 bis 12 Kohlenstoffatomen. Bevorzugt sind im allgemeinen die geradkettigen Reste mit 2 bis 7 Kohlenstoffatomen. Besonders bevorzugte Reste sind Vinyl, lE-Propenyl, lE-Butenyl, lE-Pentenyl, lE-Hexenyl und 1E-Heptenyl. Ganz besonders bevorzugt sind geradkettige Reste mit 2 bis 5 Kohlenstoffatomen.

Die Formel I umfasst z.B. die Verbindungen der allgemeinen Formeln 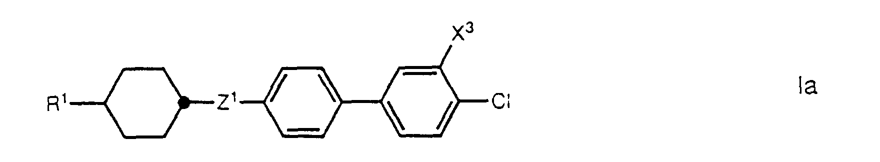 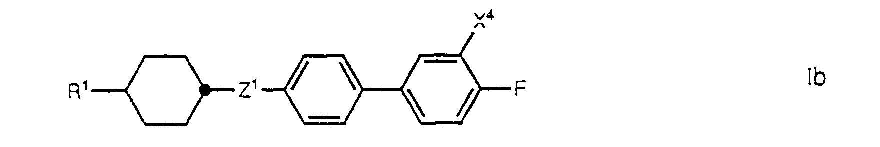 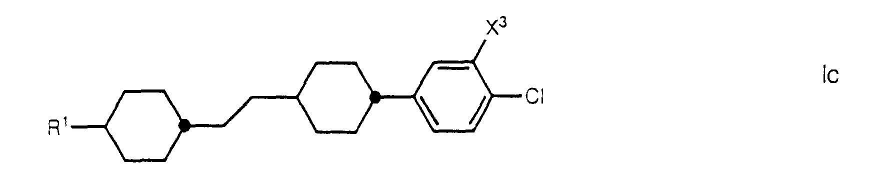 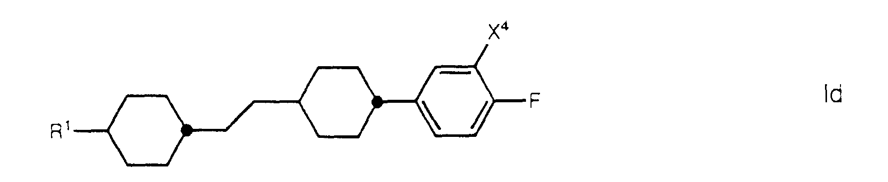 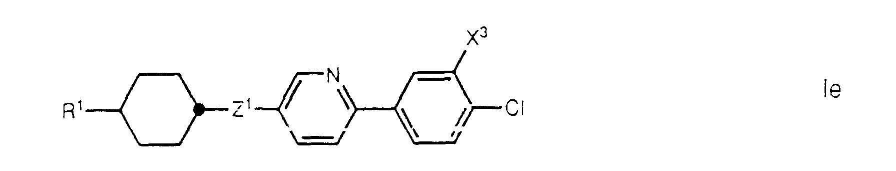 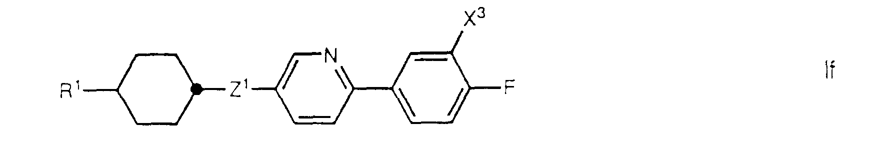 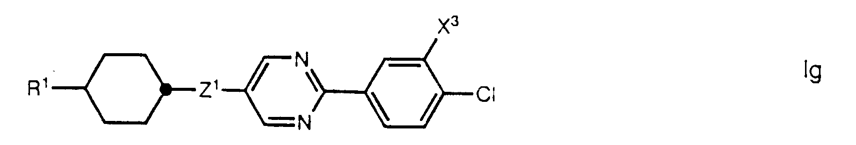 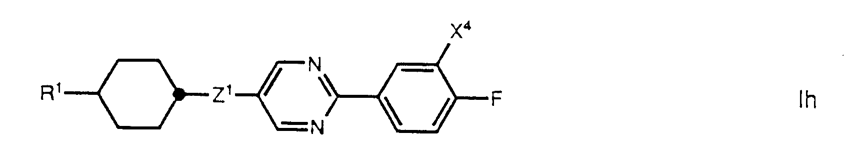 worin R¹ und Z¹ die obigen Bedeutungen haben; X³ Fluor oder Chlor bedeutet; und X⁴ Fluor oder Chlor darstellt.

Besonders bevorzugte Verbindungen der Formeln Ia, Ib und Ie-h sind Verbindungen, worin Z¹ eine einfache Kovalenzbindung darstellt.

Die Verbindungen der Formeln Ia-If können dadurch hergestellt werden, dass man einen Aldehyd der allgemeinen Formel 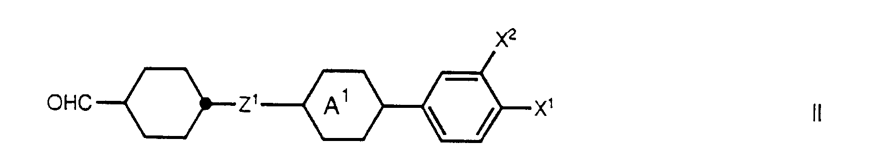 worin Z¹, X¹ und X² die oben angegebenen Bedeutungen haben und der Ring A¹ 1,4-Phenylen, Pyridin-2,5-diyl oder wenn Z¹ für -CH₂-CH₂- steht, auch trans-1,4-Cyclohexylen darstellt,
in Gegenwart einer Base mit einem geeigneten Alkyltriphenylphosphoniumhalogenid umsetzt.

Die Umsetzung mit Alkyltriphenylphosphoniumhalogenid (insbesondere mit dem Alkyltriphenylphosphoniumchlorid, -bromid oder -jodid) in Gegenwart einer organischen Base kann in an sich bekannter Weise erfolgen. Geeignete Basen sind organische Basen wie beispielsweise Kalium-tert.butylat, Natriummethylat, Natriumhydrid, Natriumamid und dergleichen. Die Reaktion wird zweckmässigerweise in einem inerten organischen Lösungsmittel, beispielsweise einem Aether, wie Diäthyläther, Tetrahydrofuran, Dioxan oder tert.Butylmethyläther durchgeführt. Temperatur und Druck sind nicht kritisch, im allgemeinen wird jedoch bei Atmosphärendruck und einer Temperatur von 0°C bis Rückflusstemperatur gearbeitet.

Wenn R¹ nicht Vinyl bedeutet, werden in der Regel E/Z-Gemische erhalten, welche nach an sich bekannten Methoden, z.B. chromatographisch auf mit Silbernitrat imprägniertem Kieselgel, getrennt werden können. Ferner können gewünschtenfalls die E/Z-Gemische bzw. die Z-Isomere durch Aequilibrierung mit Sulfinsäuren, z.B. Benzolsulfinsäure oder p-Toluolsulfinsäure, überwiegend in die E-Form übergeführt werden.

Die Aldehyde der Formel II können durch an sich bekannte Methoden hergestellt, beispielsweise nach den in den Beispielen beschriebenen Methoden.

Die Verbindungen der Formeln Ig und Ih können durch die in Schema 1 aufgezeichnete Synthese hergestellt werden. Die Herstellung von Verbindungen der allgemeinen Formel III ist bekannt und in EP-A-168683 beschrieben. Die Synthese verläuft analog zu der in Z. Naturf. 346, 1535 (1979) für strukturell verwandte Verbindungen beschriebenen. 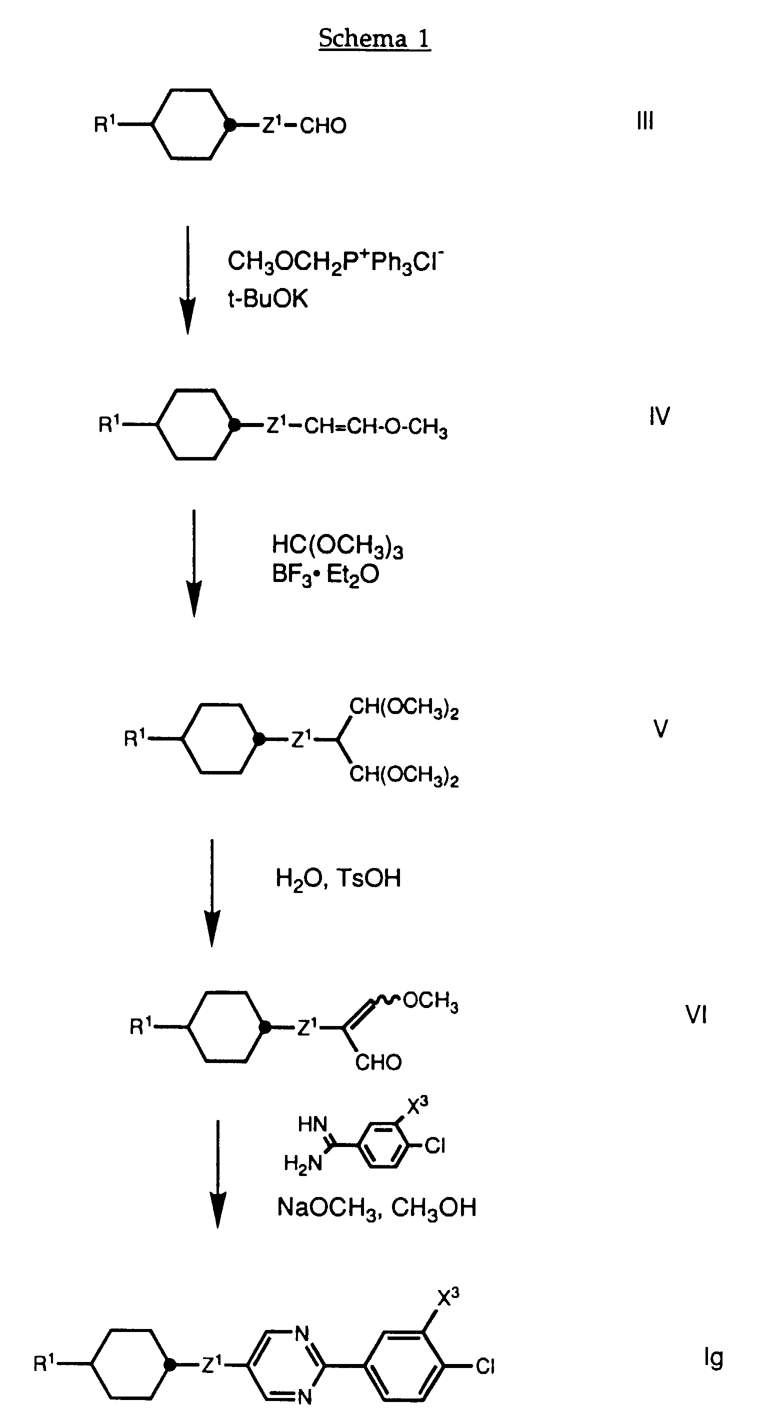 worin R¹, Z¹ und X³ die oben genannten Bedeutungen haben.

Die Verbindungen der Formel I können in Form von Gemischen untereinander und/oder mit anderen Flüssigkristallkomponenten verwendet werden, wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I oder andere Flüssigkristallkomponenten sein. Die Verbindungen der Formel I eignen sich insbesondere für nematische oder, sofern mindestens eine Komponente des Gemisches optisch aktiv ist, auch für cholesterische Gemische.

Aufgrund der guten Löslichkeit der Verbindungen der Formel I und aufgrund ihrer guten Mischbarkeit untereinander kann ihr Anteil in den erfindungsgemässen Gemischen relativ hoch sein.

Vorzugsweise enthalten die erfindungsgemässen Gemische neben einer oder mehreren Verbindungen der Formel I eine oder mehrere Verbindungen aus der Gruppe der Verbindungen der allgemeinen Formel 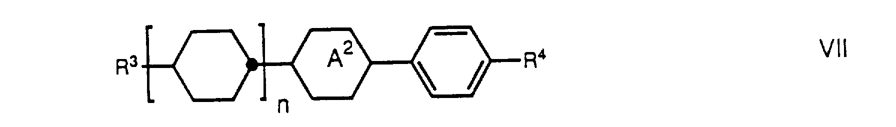 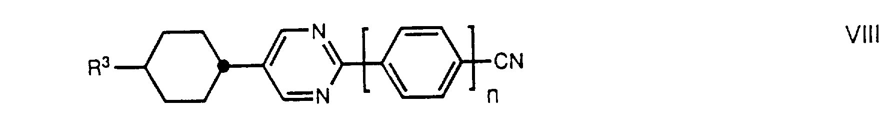 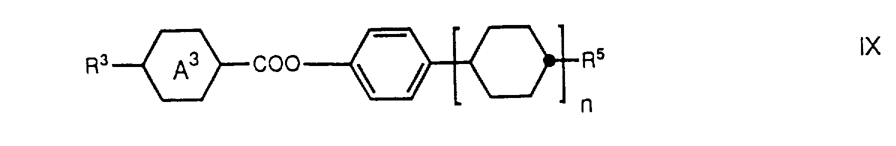 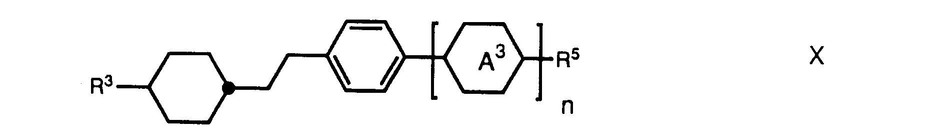 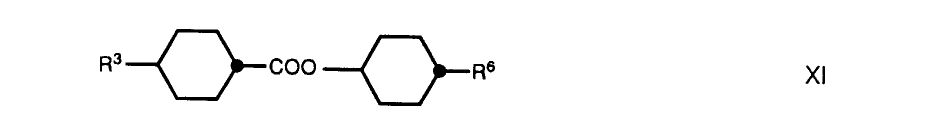 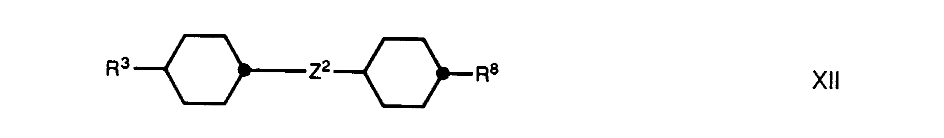 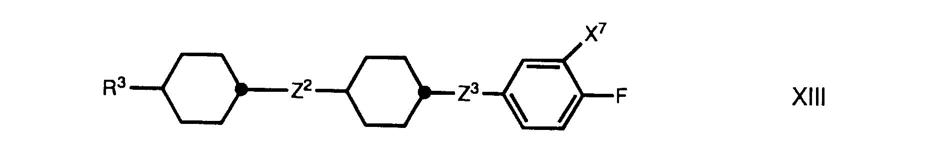 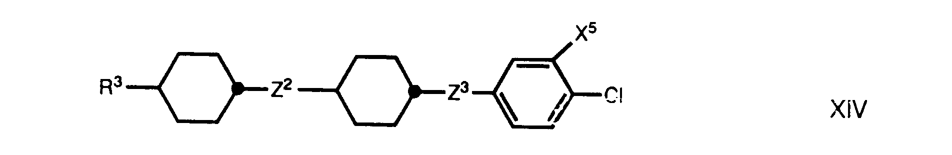 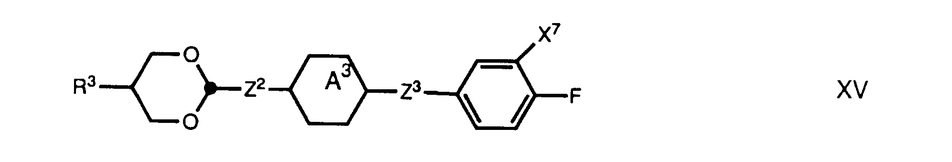 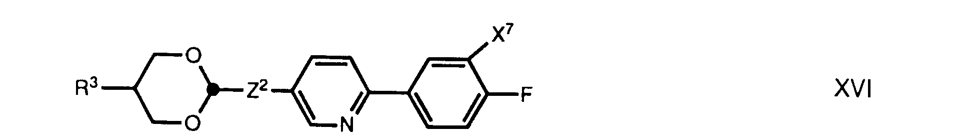 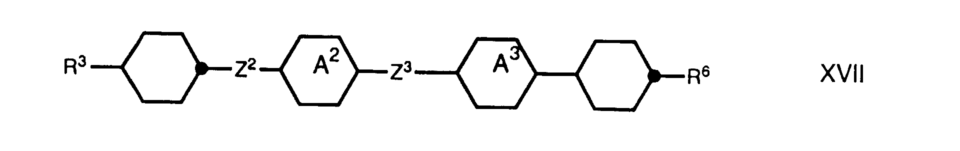 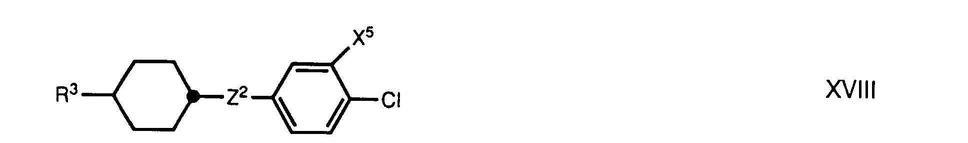 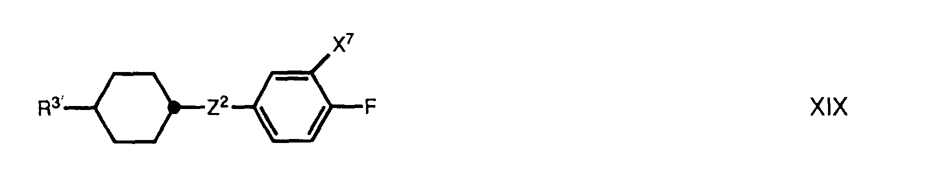 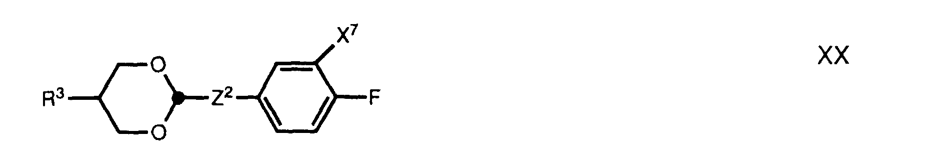 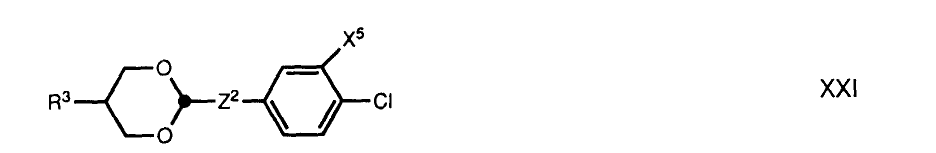 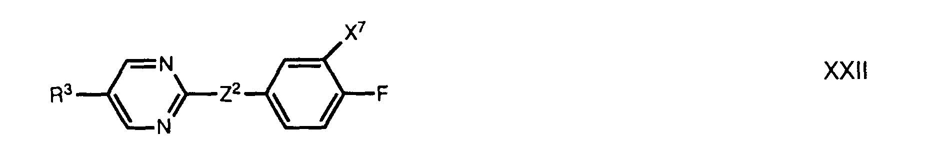 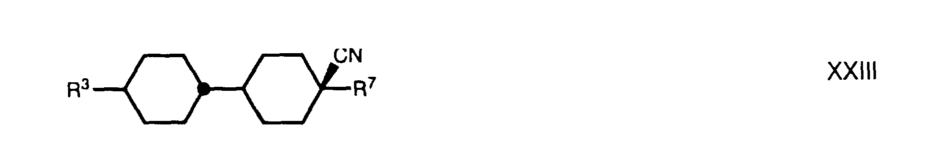 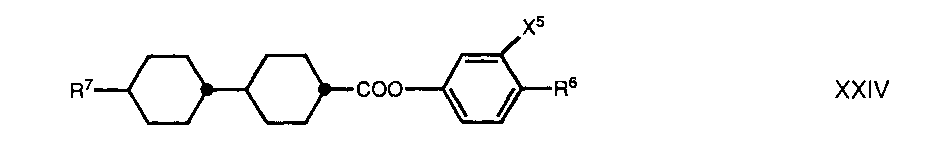 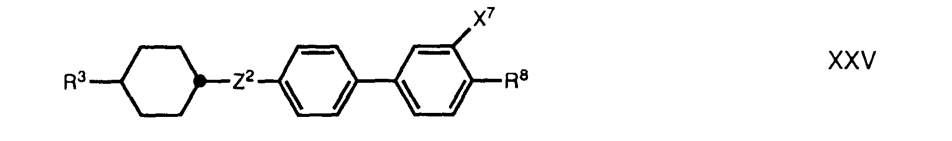 worin n für die Zahl 0 oder 1 steht; R³ und R⁶ unabhängig voneinander Alkyl, 3E-Alkenyl, 4-Alkenyl, Alkoxyalkyl oder an einem gesättigten Ring auch lE-Alkenyl bedeutet; Ring A² 1,4-Phenylen, trans-1,4-Cyclohexylen, Pyridin-2,5-diyl, Pyrimidin-2,5-diyl oder trans-1,3-Dioxan-2,5-diyl darstellt; R⁴ Cyano, -NCS, Fluor, Alkyl, 3E-Alkenyl, 4-Alkenyl, Alkoxy, 2E-Alkenyloxy, 3-Alkenyloxy oder 1-Alkinyl bezeichnet; Ring A³ 1,4-Phenylen oder trans-1,4-Cyclohexylen darstellt; R⁵ Alkyl, 3E-Alkenyl, 4-Alkenyl oder an einem Cyclohexanring auch lE-Alkenyl oder an einem Benzolring auch Cyano, -NCS, Alkoxy, 2E-Alkenyloxy oder 3-Alkenyloxy bedeutet; R⁷ Alkyl, 1E-Alkenyl, 3E-Alkenyl oder 4-Alkenyl bezeichnet; Z² und Z³ unabhängig voneinander eine einfache Kovalenzbindung oder -CH2CH2- darstellt, wobei zwei aromatische Ringe immer durch eine einfache Kovalenzbindung verknüpft sind; R⁸ Cyano, Alkyl, 3E-Alkenyl, 4-Alkenyl, Alkoxy, 2E-Alkenyloxy, 3-Alkenyloxy, Alkoxymethyl, (2E-Alkenyl)oxymethyl oder an einen Cyclohexanring auch lE-Alkenyl bedeutet; X⁵ Wasserstoff, Chlor oder Fluor bezeichnet; X⁶ Cyano, Chlor oder Fluor bedeutet; und X⁷ Wasserstoff oder Fluor bedeutet.

Der obige Ausdruck "gesättigter Ring" umfasst trans-1,4-Cyclohexylen und trans-1,3-Dioxan-2,5-diyl. Reste R³ bis R⁸ besitzen vorzugsweise je 1 bis 12 Kohlenstoffatome, besonders bevorzugt je 1 bis 7 Kohlenstoffatome. Geradkettige Reste sind im allgemeinen bevorzugt.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, S eine smektische, N eine nematische und I die isotrope Phase. V₁₀ bezeichnet die Spannung für 10% Transmission. tₒₙ und toff bezeichnen die Einschaltzeit bzw. die Ausschaltzeit. Δn bezeichnet die optische Anisotropie.

### Beispiel 1

a) Eine Suspension von 5,892 g Aethyltriphenylphosphoniumbromid in 75 ml tert.-Butylmethyläther wurde unter Rühren und Stickstoffbegasung mit 1,801 g Kalium-tert.-butylat versetzt und noch 1,5 Stunden bei Raumtemperatur gerührt. Anschliessend wurde die Suspension bei 2°C innert 45 Minuten tropfenweise mit einer Lösung von 2,269 g trans-4-(3',4'-Difluor-4-biphenylyl)cyclohexancarboxaldehyd (Herstellung gemäss Beispiel 3) in 17 ml tert.-Butylmethyläther versetzt, noch 45 Minuten gerührt und im Vakuum eingeengt. Der Rückstand (9,7 g) wurde in 50 ml Hexan aufgenommen, gerührt und filtriert. Einengen des Filtrates im Vakuum und chromatographische Reinigung des erhaltenen Rohproduktes an Kieselgel mit Methylenchlorid ergab 2,464 g 4-[trans-4-(1-Propenyl)cyclohexyl]-3',4'-difluorbiphenyl (89.7% Z; 10.1% E).
b) Eine Lösung von 2,464 g 4-[trans-4-(1-Propenyl)cyclohexyl]-3',4'-difluorbiphenyl in 32 ml Toluol wurde bei Raumtemperatur unter Rühren und Stickstoffbegasung mit 2,4 ml 3N Salzsäure und 0,375 g Benzolsulfinsäure-Natriumsalz versetzt, 4 Stunden erwärmt (Oelbadtemperatur 60-65°C), danach auf Raumtemperatur gekühlt, mit 100 ml Wasser versetzt und zweimal mit je 150 ml Diäthyläther extrahiert. Die organische Phase wurde einmal mit 50 ml 10-proz. Natriumhydrogencarbonat-Lösung und einmal mit 100 ml 10-proz. Natriumchlorid-Lösung gewaschen, über Natriumsulfat getrocknet und eingedampft. Der Rückstand (2,78g) wurde an einer mit Silbernitrat behandelten Kieselgelsäule mit Hexan und Hexan/Diäthyläther (1:1) chromatographiert und anschliessend aus Hexan kristallisiert. Dies ergab 1,195 g 4-[trans-4-(1E-Propenyl)cyclohexyl]-3',4'-difluorbiphenyl als farblose Kristalle; Smp. (C-N) 97,7-98,5°C, Klp. (N-I) 134,2-134,4°C.

Auf analoge Weise können folgende Verbindungen hergestellt werden:
4-[trans-4-(1E-Butenyl)cyclohexyl]-3',4'-difluorbiphenyl, Smp. (C-N) 74,7°C, Klp. (N-I) 120,3°C,
4-[trans-4-(1E-Pentenyl)cyclohexyl]-3',4'-difluorbiphenyl, Smp. (C-N) 72,6°C, Klp. (N-I) 124,0°C,
4-[trans-4-(1E-Propenyl)cyclohexyl]-4'-chlorbiphenyl, Smp. (C-N) 174,5°C, Klp. (N-I) 223,5°C,
4-[trans-4-(1E-Butenyl)cyclohexyl]-4'-chlorbiphenyl,
4-[trans-4-(1E-Pentenyl)cyclohexyl]-4'-chlorbiphenyl, Smp. (C-N) 166,5°C, Klp. (N-I) 214°C,
4-[trans-4-(1E-Propenyl)cyclohexyl]-3'-fluor-4'-chlorbiphenyl, Smp. (C-N) 122,4°C, Klp. (N-I) 170,6°C,
4-[trans-4-(1E-Butenyl)cyclohexyl]-3'-fluor-4'-chlorbiphenyl, Smp. (C-N) 116,9°C, Klp. (N-I) 159,3°C,
4-[trans-4-(1E-Pentenyl)cyclohexyl]-3'-fluor-4'-chlorbiphenyl, Smp. (C-N) 114°C, Klp. (N-I) 159,8°C,
4-[trans-4-(1E-Propenyl)cyclohexyl]-3'-chlor-4'-fluorbiphenyl,
4-[trans-4-(1E-Propenyl)cyclohexyl]-3',4-dichlorbiphenyl, 4-{2-[trans-4-(1E-Propenyl)cyclohexyl]äthyl}-3',4'-difluorbiphenyl,
4-{2-[trans-4-(1E-Butenyl)cyclohexyl]äthyl}-3',4'-difluorbiphenyl,
4-{2-[trans-4-(1E-Pentenyl)cyclohexyl]äthyl}-3',4'-difluorbiphenyl,
4-{2-[trans-4-(1E-Propenyl)cyclohexyl]äthyl}-4'-chlorbiphenyl,
4-{2-[trans-4-(1E-Butenyl)cyclohexyl]äthyl}-4'-chlorbiphenyl,
4-{2-[trans-4-(1E-Pentenyl)cyclohexyl]äthyl}-4'-chlorbiphenyl,
4-{2-[trans-4-(1E-Propenyl)cyclohexyl]äthyl}-3'-fluor-4'-chlorbiphenyl,
4-{2- [trans4-(1E-Butenyl)cyclohexyl]äthyl)-3'-fluor-4'-chlorbiphenyl,
4-{2-[trans-4-(1E-Pentenyl)cyclohexyl]äthyl}-3'-fluor-4'-chlorbiphenyl,
4-{2-[trans-4-(1E-Propenyl)cyclohexyl]äthyl}-3'-chlor-4'-fluorbiphenyl,
4-{2-[trans-4-(1E-Propenyl)cyclohexyl]äthyl}-3',4'-dichlorbiphenyl,
1-{trans-4-[2-[trans-4-(1E-Propenyl)cyclohexyl]äthyl]cyclohexyl}-3,4-difluorbenzol,
1-{trans-4-[2-[trans-4-(1E-Butenyl)cyclohexyl]äthyl]cyclohexyl}-3,4-difluorbenzol,
1-{trans-4-[2-[trans-4-(1E-Pentenyl)cyclohexyl]äthyl]cyclohexyl}-3,4-difluorbenzol,
1-{trans-4-[2-[trans-4-(1E-Propenyl)cyclohexyl]äthyl]cyclohexyl}-4-chlorbenzol,
1-{trans-4[2-[trans-4-(1E-Butenyl)cyclohexyl]äthyl]cyclohexyl}-4-chlorbenzol,
1-{trans-4-[2-[trans-4-(1E-Pentenyl)cyclohexyl]äthyl]cyclohexyl}-4-chlorbenzol,
1-{trans-4-[2-[trans-4-(1E-Propenyl)cyclohexyl]äthyl]cyclohexyl}-3-fluor-4-chlorbenzol,
1-{trans-4-[2-[trans-4-(1E-Butenyl)cyclohexyl]äthyl]cyclohexyl}-3-fluor-4-chlorbenzol,
1-{trans-4-[2-[trans-4-(1E-Pentenyl)cyclohexyl]äthyl]cyclohexyl}-3-fluor-4-chlorbenzol,
1-{trans-4-[2-[trans-4-(1E-Propenyl)cyclohexyl]äthyl]cyclohexyl}-3-chlor-4-fluorbenzol,
1-{trans-4-[2-[trans-4-(1E-Propenyl)cyclohexyl]äthyl]cyclohexyl}-3,4-dichlorbenzol,
2-(3,4-Difluorphenyl)-5-[trans4-(1E-propenyl)cyclohexyl]-pyridin,
2-(3,4-Difluorphenyl)-5-[trans-4-(1E-butenyl)cyclohexyl]pyridin,
2-(3,4-Difluorphenyl)-5-[trans-4-(1E-pentenyl)cyclohexyl]-pyridin,
2-(4-Chlorphenyl)-5-[trans-4-(1E-propenyl)cyclohexyl]pyridin,
2-(4-Chlorphenyl)-5-[trans-4-(1E-butenyl)cyclohexyl]pyridin,
2-(4-Chlorphenyl)-5-[trans+(1E-pentenyl)cyclohexyl]pyridin,
2-(3-Fluor-4-chlorphenyl)-5-[trans-4-(1E-propenyl)cyclohexyl]pyridin,
2-(3-Fluor-4-chlorphenyl)-5-[trans-4-(1E-butenyl)cyclohexyl]pyridin,
2-(3-Fluor-4-chlorphenyl)-5-[trans-4-(1E-pentenyl)cyclohexyl]pyridin,
2-(3-Chlor-4-fluorphenyl)-5-[trans+(1E-propenyl)cyclohexyl]pyridin,
2-(3,4-Dichlorphenyl)-5-[trans-4-(1E-propenyl)cyclohexyl]pyridin,
2-(3,4-Difluorphenyl)-5[2-[trans-4(1E-propenyl)cyclohexyl]äthyl]pyridin, Smp. (C-N) 80,4°C, Klp. (N-I) 112,8°C,
2-(3,4-Difluorphenyl)-5-[2-[trans-4-(1E-butenyl)cyclohexyl]äthyl]pyridin,
2-(3,4-Difluorphenyl-5-[2-[trans-4-(1E-pentenyl)cyclohexyl]äthyl]pyridin,
2-(4-Chlorphenyl)-5-[2-[trans-4-(1E-propenyl)cyclohexyl]äthyl]pyridin, Smp. (C-N) 137,7°C, S_{A}-N 83,7°C, Klp. (N-I) 179,5°C,
2-(4-Chlorphenyl)-5-[2-[trans-4-(1E-butenyl)cyclohexyl]äthyl]pyridin,
2-(4-Chlorphenyl)-5-[2-[trans-4-(1E-pentenyl)cyclohexyl]äthyl]pyridin,
2-(3-Fluor-4-chlorphenyl)-5-[2-[trans-4-(1E-propenyl)cyclohexyl]äthyl]pyridin,
2-(3-Fluor-4-chlorphenyl)-5-[2-[trans-4-(1E-butenyl)cyclohexyl]äthyl]pyridin,
2-(3-Fluor-4-chlorphenyl)-5-[2-[trans-4-(1E-pentenyl)cyclohexyl]äthyl]pyridin,
2-(3-Chlor-4-fluorphenyl)-5-[2-[trans-4-(1E-propenyl)cyclohexyl]äthyl]pyridin,
2-(3,4-Dichlorphenyl)-5-[2-[trans-4-(1E-propenyl)cyclohexyl]äthyl]pyridin,
2-(4-Fluorphenyl)-5-[2-[trans-4-(1E-propenyl)cyclohexyl]äthyl]pyridin, Smp. (C-N) 98°C, Klp. (N-I) 144°C,
2-(4-Fluorphenyl)-5-[2-[trans-4-(1E-butenyl)cyclohexyl]äthyl]pyridin, Smp. (C-N) 98,1°C, Klp. (N-I) 134,4°C,
2-(4-Fluorphenyl)-5-[2-[trans-4-(1E-pentenyl)cyclohexyl]äthyl]pyridin.

### Beispiel 2

Eine Suspension von 0,977 g Methyltriphenylphosphoniumbromid in 12 ml tert.-Butylmethyläther wurde unter Rühren und Stickstoffbegasung mit 0,308 g Kalium-tert.-butylat versetzt und noch 1,5 Stunden bei Raumtemperatur gerührt. Die gelbe Suspension wurde bei 2°C innert 15 Minuten tropfenweise mit einer Lösung von 0,439 g trans-4-(3',4'-Difluor-4-biphenylyl)cyclohexancarboxaldehyd in 12 ml tert.-Butylmethyläther versetzt, noch 1,5 Stunden bei Raumtemperatur gerührt und im Vakuum eingeengt. Chromatographische Reinigung des Rückstands an Kieselgel mit Methylenchlorid und anschliessende zweimalige Umkristallisation aus Hexan ergab 0,118 g reines 4-(trans-4-Vinylcyclohexyl)-3',4'-difluorbiphenyl; Smp. (C-N) 97,0°C, Klp. (N-I) 84,7°C.

Auf analoge Weise können folgende Verbindungen hergestellt werden:
4-(trans-4-Vinylcyclohexyl)-4'-chlorbiphenyl, Smp. (C-N) 165,2°C, Klp. (N-I) 183,4°C,
4-(trans-4-Vinylcyclohexyl)-4'-chlor-3'-fluorbiphenyl, Smp. (C-N) 121,9°C, Klp. (N-I) 125,3°C,
4-(trans-4-Vinylcyclohexyl)-3',4'-dichlorbiphenyl,
4-(trans-4-Vinylcyclohexyl)-3'-chlor-4'-fluorbiphenyl,
4-[2-(trans-4-Vinylcyclohexyl)äthyl]-3',4'-difluorbiphenyl,
4-[2-(trans-4-Vinylcyclohexyl)äthyl]-4'-chlorbiphenyl,
4-[2-(trans-4-Vinylcyclohexyl)äthyl]-3'-fluor-4'-chlorbiphenyl,
1-{trans-4-[2-(trans-4-Vinylcyclohexyl)äthyl]cyclohexyl}-3,4-difluorbenzol,
1-{trans-4-[2-(trans-4-Vinylcyclohexyl)äthyl]cyclohexyl}-4-chlorbenzol,
1-{trans-4-[2-(trans-4-Vinylcyclohexyl)äthyl]cyclohexyl}-3-fluor-4-chlorbenzol,
2-(3,4-Difluorphenyl)-5-(trans-4-vinylcyclohexyl)pyridin,
2-(4-Chlorphenyl)-5-(trans-4-vinylcyclohexyl)pyridin,
2-(3-Fluor-4-chlorphenyl)-5-(trans-4-vinylcyclohexyl)pyridin,
2-(3,4-Dichlorphenyl)-5-(trans-4-vinylcyclohexyl)pyridin,
2-(4-Fluorphenyl)-5-[2-(trans-4-vinylcyclohexyl)äthyl]pyridin, Smp. (C-N) 72°C, Klp. (N-I) 113,3°C,
2-(3,4-Difluorphenyl)-5-[2-(trans-4-vinylcyclohexyl)äthyl]pyridin, Smp. (C-N) 67,7°C, Klp. (N-I) 80,2°C,
2-(4-Chlorphenyl)-5-[2-(trans-4-vinylcyclohexyl)äthyl]pyridin, Smp. (C-N) 99,9°C, S_{A}-N 76,7°C, Klp. 148,7°C,
2-(3-Fluor-4-chlorphenyl)-5-[2-(trans-4-vinylcyclohexyl)äthyl]pyridin,
2-(3-Chlor-4-fluorphenyl)-5-[2-(trans-4-vinylcyclohexyl)äthyl]pyridin,
2-(3,4-Dichlorphenyl)-5-[2-(trans-4-vinylcyclohexyl)äthyl]pyridin.

Die in den Beispielen 1 und 2 als Ausgangsmaterial verwendeten Aldehyde können wie in den Beispielen 3 bis 6 beschrieben hergestellt werden.

### Beispiel 3

a) Eine Suspension von 109,6 g 4-(4-Nitrophenyl)cyclohexanon (herstellbar durch Nitrierung von 4-Phenylcyclohexanon) in 1 l Dioxan wurde mit 50 ml Triäthylamin und 2 g 5-proz. Palladium/Kohle versetzt und unter gutem Rühren bei Raumtemperatur und 0,3 bar Wasserstoffdruck hydriert. Nach 2 Stunden wurde das Gemisch filtriert. Das Filtrat wurde im Wasserstrahlvakuum bei einer Badtemperatur von 30°C eingedampft und der Eindampfrückstand über Nacht im Trockenschrank am Wasserstrahlvakuum bei 40°C getrocknet. Hierbei wurden 94,5 g 4⁻(4-Aminophenyl)cyclohexanon als weisse Kristalle mit Smp. 127-128°C erhalten.
b) In einem Sulfierkolben wurden 200 ml 4N Schwefelsäure auf 80°C erwärmt und dann mit ca. 5% einer Lösung von 37,9 g 4-(4-Aminophenyl)cyclohexanon in 200 mi 4N Schwefelsäure versetzt. Anschliessend wurden bei 80°C innert 1,5 Stunden gleichzeitig die restliche Lösung von 4-(4-Aminophenyl)cyclohexanon sowie eine Lösung von 15,2 g Natriumnitrit in 45 ml Wasser zum Reaktionsgemisch zugetropft. Danach wurde das Gemisch bei 80°C innert 30 Minuten tropfenweise mit einer Lösung von 9 g Natriumnitrit in 27 ml Wasser versetzt und noch 1 Stunde bei 80°C weitergerührt. Nach dem Abkühlen des Reaktionsgemisches auf 0°C wurden die ausgefallenen Kristalle abgenutscht, mit 200 ml kaltem Wasser gewaschen und im Trockenschrank im Wasserstrahlvakuum bei 60°C bis zur Gewichtskonstanz getrocknet. Das kristalline Rohprodukt (34,6 g) wurde in 520 ml Aethylacetat suspendiert. Die Suspension wurde 1 Stunde zum Rückfluss erhitzt, mit 1,7 g Aktivkohle versetzt und dann noch 1 Stunde zum Rückfluss erhitzt. Anschliessend wurde das Gemisch genutscht (Nachwaschen mit 40 ml warmem Aethylacetat) und das Filtrat am Wasserstrahlvakuum bei einer Badtemperatur von 40°C eingedampft. Trocknen des Eindampfrückstandes im Wasserstrahlvakuum bei 60°C bis zur Gewichtskonstanz ergab 32,2 g 4-(4-Hydroxyphenyl)cyclohexanon als gelbbraune Kristalle mit Smp. 165-166°C.
c) Eine Suspension von 13,7 g 4-(4-Hydroxyphenyl)cyclohexanon in 350 ml Aethylenchlorid wurde mit 11,6 ml Aethylenglykol und 1,6 g Amberlyst® 15 (stark saures Ionenaustauscher-Harz, Fluka AG) unter Rühren und Wasserabscheidung 3 Stunden zum Rückfluss erhitzt. Danach wurde die braune Reaktionslösung auf Raumtemperatur gekühlt und zweimal mit je 200 ml Wasser gewaschen. Die wässrigen Phasen wurden mit 200 ml Methylenchlorid nachextrahiert. Die organischen Phasen wurden über Natriumsulfat getrocknet, filtriert und am Rotationsverdampfer eingeengt. Die erhaltenen braunen Kristalle (17,4 g) wurden in 50 ml Aethylacetat heiss gelöst. Die Lösung wurde mit 50 ml Hexan versetzt und im Wasserbad kristallisieren gelassen. Der Niederschlag wurde abgenutscht und bei 50°C im Vakuum getrocknet. wobei 12,4 g Rohprodukt als braune Kristalle erhalten wurden. Aufarbeitung der Mutterlauge ergab weitere 4,8 g Rohprodukt. Das Rohprodukt wurde an Kieselgel mit Methylenchlorid/Aceton (Volumenverhältnis zuerst 97:3, dann 95:5) bei 0,4 bar chromatographisch gereinigt. Umkristallisation des orangen, kristallinen Produktes aus Aethylacetat/Hexan (Vol. 1:2) ergab 15,0 g 4-(1,4-Dioxa-8-spiro[4.5]decyl)phenol als gelborange Kristalle mit Smp. 151,7-152,6°C.
d) Eine Suspension von 13,0 g 4-(1,4-Dioxa-8-spiro[4.5]decyl)phenol in 150 ml Methylenchlorid wurde mit 7,6 ml 2,6-Lutidin versetzt, wobei der Niederschlag in Lösung ging. Die orange Lösung wurde auf 0°C gekühlt und innert 15 Minuten bei 0-2°C tropfenweise mit einer Lösung von 10,9 ml Trifluormethan-sulfonsäureanhydrid in 80 ml Methylenchlorid versetzt. Das Reaktionsgemisch wurde noch 30 Minuten bei 0-2°C weitergerührt und dann bei 0-5°C innert 5 Minuten tropfenweise mit 100 ml Wasser versetzt. Anschlies-send wurde die organische Phase mit 100 ml ca. 10-proz. Kupfersulfat-Lösung gewaschen. Die wässrigen Phasen wurden mit 100 ml Methylenchlorid extrahiert. Die organischen Phasen wurden über Natriumsulfat getrocknet, filtriert und am Rotationsverdampfer eingeengt. Das erhaltene bräunliche Oel (20,1 g) wurde an Kieselgel mit Methylenchlorid bei 0,4 bar chromatographisch gereinigt. Hierbei wurden 9,7 g 4-(1,4-Dioxa-spiro[4.5]decyl)phenyl-trifluormethansulfonat als bräunliches, kristallisierendes Oel erhalten. Weitere Fraktionen ergaben 9,2 g hräunliches Oel (bestehend aus 70% des gewünschten Ketals und 30% des entsprechenden Ketons), welches nicht aufgearbeitet wurde.
e) Ein Gemisch von 0,399 g Magnesium-Spänen, einem Körnchen Jod und 10 ml Diäthyläther wurde unter Rühren und Stickstoffbegasung innert 15 Minuten tropfenweise mit einer Lösung von 1,88 ml 3,4-Difluor-1-brombenzol in 20 ml Diäthyläther versetzt. Das Gemisch wurde leicht erwärmt und dann 15 Minuten am Rückfluss gerührt. Die erhaltene Grignard-Reagens-Lösung wurde auf Raumtemperatur gekühlt und filtriert. Ein Gemisch von 2,0 g 4-(1,4-Dioxa-8-spiro[4.5]decyl)phenyl-trifluormethansulfonat, 20 ml Tetrahydrofuran und 0,5 g Tetrakis(triphenylphosphin)palladium wurde unter Rühren und Stickstoffbegasung auf 60-65°C erwärmt und innert 4 Stunden tropfenweise mit der Grignard-Reagens-Lösung versetzt. Das Reaktionsgemisch wurde anschliessend noch 2,5 Stunden bei 60-65°C weitergerührt, während dieser Zeit wurde der Aether durch 10 ml Tetrahydrofuran ersetzt, die dunkle Lösung wurde 18 Stunden bei 65-67°C gerührt, auf 10-15°C gekühlt, vorsichtig mit 30 ml Wasser und dann tropfenweise mit 5 ml 3N Salzsäure versetzt. Das Gemisch wurde zweimal mit je 100 ml Diäthyläther extrahiert. Die Aetherphasen wurden mit 100 ml 10-proz. Natriumhydrogencarbonat-Lösung und mit 100 ml 10-proz. Kochsalzlösung gewaschen, über Natriumsulfat getrocknet, filtriert und ergaben nach Einengen des Filtrates 3,13 g rohes 4-(1,4-Dioxa-8-spiro[4.5]decyl)-3',4'-difluorbiphenyl. Chromatographische Reinigung an 120 g Kieselgel mit Aethylacetat/Hexan (1:9) lieferte 1,5 g bräunliche Kristalle.
f) Eine Lösung von 1,5 g 4-(1,4-Dioxa-8-spiro[4.5]decyl)-3',4'-difluorbiphenyl in 50 ml Toluol wurde mit 15 ml Ameisensäure 1 Stunde bei Raumtemperatur gerührt, mit 100 ml Wasser versetzt und zweimal mit je 100 ml Diäthyläther extrahiert. Die organischen Phasen wurden vereinigt, je einmal mit 100 ml 10-proz. Natriumhydrogencarbonat-Lösung und 100 ml 10-proz. Natriumchlorid-Lösung gewaschen, über Natriumsulfat getrocknet und ergaben nach dem Eindampfen 1,402 g bräunlichen Rückstand. Eine chromatographische Reinigung des Rückstandes an 60 g Kieselgel mit Methylenchlorid ergab 1,307 g 4-(3',4'-Difluor-4-biphenylyl)cyclohexanon als bräunliche Kristalle, welche nach Umkristallisation aus Methylenchlorid/Hexan 1,038 g farblose Kristalle ergaben.
g) Eine mit Stickstoff begaste Suspension von 5,707 g trockenem (Methoxymethyl)triphenylphosphoniumchlorid in 35 ml tert.-Butylmethyläther wurde unter Rühren bei -15°C mit 1,953 g Kalium-tert.-butylat versetzt, 30 Minuten gerührt und auf -5°C erwärmt. Zu der orangen Suspension wurde innert 10 Minuten 3,185 g 4-(3',4'-Difluor-4-biphenylyl)cyclohexanon in 20 ml Tetrahydrofuran (absolut) und 10 ml tert.-Butylmethyläther getropft, das Kühlbad wurde entfernt, das Reaktionsgemisch 1,5 Stunden bei Raumtemperatur gerührt, mit 50 ml Hexan verdünnt, filtriert und der Filterrückstand mit Hexan gewaschen. Das Filtrat ergab nach Eindampfen 6,2 g braune ölige Kristalle. Der Filterrückstand wurde in 25 ml Methylenchlorid und 50 ml Hexan aufgenommen und filtriert. Dieses Filtrat ergab weitere 1,8 g Produkt als braune ölige Kristalle. Das Produkt (6,2 und 1,8 g) ergab nach chromatographischer Reinigung an 120 g Kieselgel bei einem Druck von 0,5 bar mit Methylenchlorid 3,552 g 4-[(Methoxymethyliden)cyclohexyl]-3',4'-difluorbiphenyl als farbloses Oel.
h) Ein Gemisch von 3,552 g 4-[(Methoxymethyliden)cyclohexyl]-3',4'-difluorbiphenyl in 18 ml Tetrahydrofuran und 4,5 ml 2N Salzsäure wurde unter Stickstoffbegasung 30 Minuten zum Sieden erhitzt, abgekühlt, mit 150 ml Wasser versetzt und zweimal mit je 100 ml Methylenchlorid extrahiert. Die organischen Phasen wurden vereinigt, mit 100 ml 10-proz. Natriumchlorid-Lösung gewaschen, über Natriumsulfat getrocknet und eingedampft. Dies ergab 3,418 g eines cis/trans-Gemisches von 4-(4-Formylcyclohexyl)-3',4'-difluorbiphenyl (15,1% cis/84,8% trans), das in 80 ml Methanol und 8 ml Methylenchlorid gelöst und unter Stickstoff mit 3 Tropfen Triäthylamin und 1,6 ml 20-proz. Natronlauge (g/g) 30 Minuten gerührt wurde. Die gelbliche Lösung wurde mit 150 ml Wasser versetzt und zweimal mit je 100 ml Methylenchlorid extrahiert. Die organische Phase wurde mit 100 ml 10-proz. Natriumclorid-Lösung gewaschen, über Natriumsulfat getrocknet und ergab nach dem Eindampfen 3,398 g farbloses Oel, welches nach chromatographischer Reinigung an 120 g Kieselgel bei einem Druck von 0,5 bar mit Hexan/Aethylacetat (9:1) und anschliessender Kristallisation aus Methylenchlorid/Hexan bei 0°C 2,629 g reinen trans-4-(3',4'-Difluor-4-biphenylyl)cyclohexancarboxaldehyd als farblose Kristalle lieferte.

Auf analoge Weise können folgende Verbindungen hergestellt werden:
trans-4-(4'-Chlor-4-biphenylyl)carboxaldehyd,
trans-4-(3',4'-Dichlor-4-biphenylyl)carboxaldehyd,
trans-4-(3'-Chlor-4'-fluor-4-biphenylyl)carboxaldehyd,
trans-4-(4'-Chlor-3'-fluor-4-biphenylyl)carboxaldehyd.

### Beispiel 4

a) 4,53 g Magnesium-Späne und ein Körnchen Jod wird unter Rühren und Stickstoffbegasung mit 150 ml Tetrahydrofuran versetzt. Anschliessend wird das Gemisch innert 20 Minuten tropfenweise mit einer Lösung von 38,4 g 1-Brom-4-chlorbenzol in 150 ml Tetrahydrofuran versetzt und 1,25 Stunden am Rückfluss gekocht. Danach wird das Reaktionsgemisch auf 0°C gekühlt und innert 30 Minuten bei 0-5°C tropfenweise mit einer Lösung von 35,7 g 8-[2-(4-Oxocyclohexyl)äthyl]-1,4-dioxaspiro[4.5]decan (Herstellung ausgehend von 4,4'-(1,2-Aethandiyl)bisphenol in Analogie zu der in EP-A-310'067 beschriebenen Synthese von 8-(4-Oxocyclohexyl)-1,4-dioxaspiro[4.5]decan) in 240 ml Tetrahydrofuran versetzt. Das Reaktionsgemisch wird noch 4 Stunden ohne Kühlung gerührt, dann innert 10 Minuten mit 240 ml 10-proz. Ammoniumchlorid-Lösung versetzt und mit Diäthyläther extrahiert. Die AetherPhasen werden mit Wasser gewaschen, über Natriumsulfat getrocknet, filtriert und eingeengt, wobei 1-(4-Chlorphenyl)-4-[2-(1,4-dioxa-spiro[4.5]decyl)äthyl]cyclohexanol erhalten wird.
b) Eine Lösung von 54,7 g 1-(4-Chlorphenyl-4-[2-(1,4-dioxa-spiro[4.5]decyl)äthyl]cyclohexanol in 570 ml Aethylenchlorid wird mit 6,95 ml Aethylenglykol und 6,95 g Amberlyst® 15 (stark saures Ionenaustauscher-Harz, Fluka AG) versetzt und 2,3 Stunden durch neutrales Aluminiumoxid rückflussiert. Danach wird das Reaktionsgemisch auf Raumtemperatur gekühlt, filtriert und mit Wasser gewaschen. Die wässrigen Phasen werden mit Methylenchlorid extrahiert. Die organischen Phasen werden über Natriumsulfat getrocknet, filtriert und eingeengt. Das erhaltene Rohprodukt wird in 150 ml Aethylacetat gelöst. Die Lösung wird mit Aktivkohle versetzt und warm filtriert. Das Filtrat wird teilweise eingedampft, dann mit Methanol versetzt und bei Raumtemperatur aufbewahrt. Hierbei wird 4-Chlor-1-[4-(2-(1,4-dioxa-8-spiro[4.5]decyl)-äthyl)-1-cyclohexenyl]benzol als farbloser Niederschlag erhalten.
c) Eine Lösung von 35,0 g 4-Chlor-1-[4-(2-(1,4-dioxa-8-spiro[4.5]decyl)äthyl)-1-cyclohexenyl]benzol in 1 Toluol wird mit 2,6 g Palladium/Kohle (10%) versetzt und bei Raumtemperatur unter Normaldruck bis zum Stillstand der Wasserstoffaufnahme hydriert. Danach wird das Reaktionsgemisch filtriert und das Filtrat eingeengt, wobei rohes 4-Chlor-1-[4-(2-(1,4-dioxa-8-spiro[4.5]-decyl)äthyl)cyclohexyl]benzol erhalten wird. Eine Suspension von 32,8 g Aluminiumchlorid in 215 ml Methylenchlorid wird bei -18°C unter Rühren und Stickstoffbegasung innert 10 Minuten mit einer Lösung des erhaltenen Rohproduktes in 110 ml Methylenchlorid versetzt und noch 30 Minuten bei 0°C gerührt. Anschliessend wird das Reaktionsgemisch auf 600 ml Eis/Wasser gegossen, 10 Minuten gerührt und dann dreimal mit Methylenchlorid extrahiert. Die organischen Phasen werden nacheinander mit Wasser, mit gesättigter Natriumhydrogencarbonat-Lösung und nochmals mit Wasser gewaschen, dann über Natriumsulfat getrocknet und filtriert. Einengen des Filtrates ergibt 4-Chlor-1-[trans-4-[2-(1,4-dioxa-8-spiro[4.5]decyl)äthyl]cyclohexyl]benzol.
d) Ein Gemisch von 31,1 g 4-Chlor-1-[trans-4-(2-(1,4-dioxa-8-spiro[4.5]decyl)äthyl)cyclohexyl]benzol, 207 ml Toluol und 103 ml Ameisensäure wird bei Raumtemperatur unter Stickstoffbegasung 1,25 Stunden gerührt. Danach wird das Reaktionsgemisch auf 500 ml Wasser gegossen und mit Methylenchlorid extrahiert. Die organischen Phasen werden mit gesättigter Natriumhydrogencarbonat-Lösung und mit Wasser gewaschen, über Natriumsulfat getrocknet und filtriert. Einengen des Filtrates ergibt 4-[2-[trans-4-(4-Chlorphenyl)cyclohexyl]äthyl]cyclohexanon.
e) Eine Suspension von 35,5 g (Methoxymethyl)triphenylphosphoniumchlorid in 210 ml tert.Butylmethyläther wird bei -15°C unter Rühren und Stickstoffbegasung mit 12,2 g Kalium-tert.-butylat versetzt. Die Suspension wird noch 30 Minuten bei 5°C gerührt, dann bei 0-5°C innert 40 Minuten tropfenweise mit einer Lösung von 20 g 4-[2-[trans-4-(4-Chlorphenyl)cyclohexyl]äthyl]cyclohexanon in 50 ml Tetrahydrofuran und 200 ml tert.-Butylmethyläther versetzt und noch 1 Stunde bei Raumtemperatur gerührt. Anschliessend wird das Reaktionsgemisch genutscht und das Filtrat eingeengt. Das erhaltene Rohprodukt wird mit 250 ml Hexan versetzt. Das Gemisch wird 10 Minuten bei Raumtemperatur gerührt und genutscht. Einengen des Filtrates und chromatographische Reinigung des erhaltenen Produktes an Kieselgel mit Hexan und Hexan/Aethylacetat ergibt schliesslich 4-Chlor-1-[trans-4-[2-(4-methoxymethyliden)cyclohexyl)äthyl]cyclohexyl]benzol.
f) Eine Lösung von 18,1 g 4-Chlor-1-[trans-4-[2-(4-methoxymethyliden)cyclohexyl)äthyl]cyclohexyl]benzol in 90 ml Tetrahydrofuran wird mit 22,5 ml 2N Salzsäure versetzt und unter Rühren und Stickstoffbegasung 30 Minuten am Rückfluss gekocht. Anschliessend wird das Reaktionsgemisch auf Raumtemperatur gekühlt, auf 400 ml Wasser gegossen und mit Methylenchlorid extrahiert. Die organischen Phasen werden mit Wasser gewaschen, über Natriumsulfat getrocknet, filtriert und eingeengt. Der Rückstand wird in 150 ml tert.-Butylmethyläther gelöst. Die Lösung wird weitgehend eingedampft und dann mit 200 ml Methanol versetzt. Kristallisation bei -25°C und Umkristallisation des farblosen, kristallinen Niederschlages ergibt reinen trans-4-[2-(trans-4-(4-Chlorphenyl)cyclohexyl)äthyl]cyclohexancarboxaldehyd.

In analoger Weise können folgende Verbindungen hergestellt werden:
trans-4-{2-[trans-4-(3,4-Difluorphenyl)cyclohexyl]äthyl}cyclohexancarboxaldehyd,
trans-4-{2-[trans-4-(3-Fluor-4-chlorphenyl)cyclohexyl]äthyl}cyclohexancarboxaldehyd,
trans-4-{2-[trans-4-(3,4-Dichlorphenyl)cyclohexyl]äthyl}cyclohexancarboxaldehyd.

### Beispiel 5

a) Zu einer Lösung von 72,8 ml Triisopropylborat in 200 ml Tetrahydrofuran wird bei -65°C unter Stickstoff eine aus 46,8 g 1-Brom-3,4-difluorbenzol und 6 g Magnesium in 150 ml Tetrahydrofuran zubereitete Grignard-Reagens-Lösung getropft. Das Gemisch wird 1 Stunde bei -65°C gerührt, auf 15-20°C erwärmt, tropfenweise mit 100 ml 10-proz. (V/V) Schwefelsäure versetzt, über Nacht stehengelassen und vom ausgefallenen Salz abfiltriert. Die wässrige Phase des Filtrates wird abgetrennt und mit Diäthyläther extrahiert. Die organischen Phasen werden vereinigt, mit Natriumchlorid-Lösung gewaschen, über Natriumsulfat getrocknet und eingedampft. Der Rückstand ergibt nach Auskochen mit Hcxan 22,1 g farblose 3,4-Difluorphenylborsäure, Smp. 275-277,5°C (sublimiert).
b) Ein Gemisch aus 6,2 g 3,4-Difluorphenylborsäure, 8,5 g 2,5-Dibrompyridin, 0,31 g Tetrakistriphenylphosphinpalladium, 60 ml Aethanol, 120 ml Benzol und 120 ml 2N Natriumcarbonat-Lösung wird 7 Stunden unter Rühren zum Sieden erhitzt (66°C). Nach dem Erkalten wird mit Diäthyläther extrahiert. Die organische Phase wird mit 100 ml gesättigter Natriumhydrogencarbonat-Lösung und 100 ml Wasser gewaschen, über Natriumsulfit getrocknet, filtriert und eingeengt. Chromatographische Reinigung des Rückstands an Kieselgel mit Hexan/Aethylacetat 19:1 liefert reines 5-Brom-2-(3,4-difluorphenyl)pyridin.
c) Eine Lösung von 10,62 g 4-Chlorcyclohexancarboxaldehyd in 150 ml Toluol wird mit 11,5 ml 1,3-Propandiol und 1 g Amberlyst® 15 versetzt und unter Rühren 2 Stunden am Wasserabscheider zum Sieden erhitzt. Das Reaktionsgemisch wird mit 2 ml Triäthylamin versetzt und nach dem Erkalten filtriert. Das Filtrat wird mit dreimal 30 ml Wasser gewaschen, über Natriumsulfat getrocknet und eingeengt. Man erhält 2-(4-Chlorcyclohexyl)-1,3-dioxan.
d) Ein aus 12,28 g 2-(4-Chlorcyclohexyl)-1,3-dioxan und 1,45 g Magnesium in 70 ml Diäthyläther hergestelltes Grignard-Reagens wird mit 250 ml Tetrahydrofuran und 4,3 g Kupfer(I)bromid in Stickstoffatmosphäre 30 Minuten bei -78°C gerührt. Dann wird eine Lösung von 4,06 g 5-Brom-2-(3,4-difluorphenyl)pyridin (aus Stufe b) in 30 ml Tetrahydrofuran zugetropft. Das Reaktionsgemisch wird noch 2 Stunden bei -78°C und über Nacht ohne Bad gerührt, tropfenweise mit 20 ml konz. Ammoniak versetzt und mit 50 ml Wasser verdünnt. Die wässrige Phase wird abgetrennt und mit 50 ml Diäthyläther extrahiert. Die vereinigte organische Phase wird dreimal mit je 50 ml Kochsalzlösung gewaschen, über Natriumsulfat getrocknet, filtriert und eingeengt. Chromatographie des Rückstandes an Kieselgel mit Hexan/Aethylacetat 9:1 liefert 2-(3,4-Difluorphenyl)-5-[4-(1,3-dioxan-2-yl)cyclohexyl]pyridin.
e) Ein Gemisch aus 1,65 g 2-(3,4-Difluorphenyl)-5-[4-(1,3-dioxan-2-yl)cyclohexyl]pyridin, 15 ml Toluol und 10 ml Ameisensäure wird 10 Stunden bei Raumtemperatur gerührt und anschliessend vorsichtig mit gesättigter Natriumcarbonat-Lösung bis pH = 7 neutralisiert. Die wässrige Phase wird abgetrennt und zweimal mit Toluol extrahiert, die organischen Phasen werden vereinigt, mit Wasser gewaschen, über Natriumsulfat getrocknet und eingedampft. Der Rückstand wird in 15 ml Methanol gelöst und in 10 ml, auf 0°C gekühlte, 0,1N methanolische Kaliumhydroxid-Lösung getropft. Das Gemisch wird 1 Stunde bei 0°C gerührt, auf 40 ml Eiswasser gegossen und dreimal mit je 25 ml Diäthyläther extrahiert. Die organischen Phasen werden vereinigt, mit Wasser neutral gewaschen, über Natriumsulfat getrocknet und ergeben nach dem Eindampfen trans-4-[2-(3,4-Difluorphenyl)-5-pyridyl]cyclohexancarboxaldehyd (cis-Anteil ca. 8%).

Analog können folgende Verbindungen hergestellt werden:
trans-4-[2-(3-Chlor-4-fluorphenyl)-5-pyridyl]cyclohexancarboxaldehyd,
trans-4-[2-(4-Chlorphenyl)-5-pyridyl]cyclohexancarboxaldehyd,
trans-4-[2-(3,4-Dichlorphenyl)-5-pyridyl]cyclohexancarboxaldehyd,
trans-4-[2-(4-Chlor-3-fluorphenyl)-5-pyridyl]cyclohexancarboxaldehyd.

### Beispiel 6

a) Ein Gemisch aus 85 g 6-Chlor-3-pyridincarbinol, 800 ml Dioxan und 129,8 g 3,4-Dihydro-2H-pyran wurde mit 5,7 g p-Toluolsulfonsäure-Monohydrat versetzt, 0,75 Stunden bei Raumtemperatur und anschliessend 2,75 Stunden bei 65°C gerührt. Das Reaktionsgemisch wurde mit 15 ml Triäthylamin versetzt und nach dem Abkühlen in Diäthyläther aufgenommen. Mehrmaliges Waschen mit gesättigter Kochsalz-Lösung, Trocknen über Natriumsulfat und Eindampfen lieferte 153,2 g rohes 2-Chlor-5-[(tetrahydro-2-pyranyloxy)methyl]pyridin als braunrotes Oel.
b) Ein Zweiphasen-Gemisch aus 5,7 g 3,4-Difluorphenylborsäure, 7,5 g rohem 2-Chlor-5-[(tetrahydro-2-pyranyloxy)methyl]pyridin, 0,38 g Tetrakis(triphenylphosphin)palladium, 60 ml Aethanol, 120 ml Benzol und 120 ml 2N Natriumcarbonat-Lösung wurde unter starkem Rühren 7 Stunden zum Sieden erhitzt. Nach dem Erkalten wurden mit 200 ml Diäthyläther verdünnt, die Phasen getrennt und die organische Phase mit je 100 mi gesättigter Natriumhydrogencarbonat und Wasser gewaschen. Die organische Phase wurde zweimal mit je 50 ml 3N Salzsäure extrahiert. Die saure wässrige Lösung wurde nach 45 Minuten stehengelassen, mit verdünnter Natronlauge alkalisch gestellt und das Produkt mit Diäthyläther extrahiert. Die organische Phase wurde neutral gewaschen, über Natriumsulfat getrocknet und lieferte nach Eindampfen 6,1 g festes, reines 6-(3,4-Difluorphenyl)-3-pyridincarbinol.
c) Im Eisbad werden 0,3 ml Thionylchlorid zu einer Lösung von 0,5 g 6-(3,4-Difluorphenyl)-3-pyridincarbinol in 20 ml Dichlormethan getropft. Das Reaktionsgemisch wurde 0,75 Stunden bei 5°C und 1,5 Stunden bei Raumtemperatur gerührt, dann tropfenweise mit 5 ml gesättigter Natriumcarbonat-Lösung versetzt. Die Wasserphase (pH 8) wurde abgetrennt und mit Dichlormethan extrahiert. Die vereinigte organische Phase wurde mit Wasser gewaschen, über Natriumsulfat getrocknet und eingeengt. Es resultierten 0,46 g reines 5-Chlormethyl-2-(3,4-difluorphenyl)pyridin.
d) Ein Gemisch von 0,46 g 5-Dichlormethyl-2-(3,4-difluorphenyl)pyridin, 0,6 g Triphenylphosphin und 10 ml Xylol wurde 24 Stunden zum Sieden erhitzt. Nach dem Erkalten wurde der Niederschlag abfiltriert, mit Toluol gewaschen und getrocknet. Es resultierten 0,48 g farbloses [2-(3,4-Difluorphenyl)-5-pyridyl]methyltriphenylphosphoniumchlorid, Smp. 259-260,5°C.
e) Eine Suspension von 12,05 g [2-(3,4-Difluorphenyl)-5-pyridyl]methyltriphenylphosphoniumchlorid in 70 ml Diäthyläther wird in Stickstoffatmosphäre mit 2,67 g Kalium-tert.-butylat versetzt. Die dunkelgelbe Suspension wird noch 30 Minuten bei Raumtemperatur gerührt. Bei 0°C wird dann eine Lösung von 2,74 g trans-4-Formylcyclohexancarbonitril in 30 ml Diäthyläther zugetropft. Das Reaktionsgemisch wird noch 1 Stunde bei 0°C gerührt, dann mit 40 ml IN Natriumhydrogencarbonat-Lösung verrührt, mit Diäthyläther verdünnt. Die organische Phase wird abgetrennt, mit Wasser neutral gewaschen, über Natriumsulfat getrocknet und eingeengt. Der Rückstand wird an Kieselgel mit Hexan/Aethylacetat 3:1 (v/v) chromatographiert. Man erhält trans-4-[2-[2-(3,4-Difluorphenyl)-5-pyridyl]vinyl]cyclohexancarbonitril.
f) Eine Lösung von 4,9 g trans-4-[2-[2-(3,4-Difluorphenyl)-5-pyridyl]vinyl]cyclohexancarbonitril in 120 ml Toluol wird mit 0,5 g 10-proz. Palladiumkohle bei Raumtemperatur und Normaldruck hydriert. Filtration und Eindampfen des Filtrates liefert trans-4-[2-[2-(3,4-Difluoqhenyl)-5-pyridyl]äthyl]cyclohexancarbonitril.
g) Eine Lösung von 4,8 g trans-4-[2-(2-[3,4-Difluorphenyl]-5-pyridyl)äthyl]cyclohexancarbonitril in 150 ml Toluol wird bei -78°C unter Stickstoff mit einer Lösung von 24 ml 1,2M Diisobutylaluminiumhydrid in Toluol versetzt. Das Reaktionsgemisch wird 1 Stunde bei -78°C gerührt, auf 200 ml gesättigte Ammoniumchlorid-Lösung gegossen. Die wässrige Phase wird abgetrennt und zweimal mit Toluol gewaschen. Die organischen Phasen werden vereinigt, mit Wasser gewaschen, über Natriumsulfat getrocknet und eingedampft. Man erhält trans-4-[2-(2-[3,4-Difluorphenyl]-5-pyridyl)äthyl]cyclohexancarboxaldehyd.

Auf analoge Weise können folgende Verbindungen hergestellt werden:
trans-4-[2-(2-[4-Fluorphenyl]-5-pyridyl)äthyl]cyclohexancarboxaldehyd,
trans-4-[2-(2-[3-Chlor-4-fluorphenyl]-5-pyridyl)äthyl]cyclohexancarboxaldehyd,
trans-4-[2-(2-[4-Chlorphenyl]-5-pyridyl)äthyl]cyclohexancarboxaldehyd,
trans-4-[2-(2-[3,4-Dichlorphenyl]-5-pyridyl)äthyl]cyclohexancarboxaldehyd,
trans-4-[2-(2-[4-Chlor-3-fluorphenyl]-5-pyridyl)äthyl]cyclohexancarboxaldehyd.

### Beispiel 7

Die Pyrimidinverbindungen Ig und Ih können auf folgende Weise hergestellt werden:
a) Eine Suspension von 21 g wasserfreiem (Methoxymethyl)triphenylphosphoniumchlorid in 100 ml abs. Diäthyläther wird bei 0°C in Stickstoffatmosphäre mit 6,86 g Kalium-tert.-butylat versetzt. Die rote Suspension wird 15 Minuten bei 0°C gerührt und dann tropfenweise bei 5-10°C mit einer Lösung von 7,9 g trans-4-(1E-Pentenyl)cyclohexancarboxaldehyd in 30 ml Diäthyläther versetzt. Das Gemisch wird 4 Stunden bei Raumtemperatur gerührt, mit 40 ml eiskalter gesättigter Natriumhydrogencarbonat-Lösung und zweimal je 50 ml Wasser gewaschen, über Natriumsulfat getrocknet, filtriert und eingeengt. Der Rückstand wird in 100 ml Hexan und 40 ml 80-proz. wässrigen Methanol gelöst. Die Hexanphase wird mit 10 ml 80-proz. wässrigen Methanol und 20 ml Wasser gewaschen, über Natriumsulfat getrocknet und eingeengt. Destillation des Rückstands im Vakuum liefert trans-1-(2-Methoxyvinyl)-4-(1E-pentenyl)cyclohexan als farbloses Oel.
b) 20 ml Orthoameisensäuretrimethylester werden in Stickstoffatmosphäre bei 0-5°C mit 0,38 ml Bortrifluorid-diäthylätherat und dann tropfenweise mit 7,30 ml trans-1-(2-Methoxyvinyl)-4-(1E-pentenyl)cyclohexan versetzt. Das Reaktionsgemisch wird 3 Stunden bei 3°C stehengelassen, mit 0,38 ml Triäthanolamin versetzt und im Vakuum eingeengt. Der Rückstand wird in 50 ml Hexan gelöst, die Lösung mit 10 ml gesättigter Natriumhydrogencarbonat-Lösung und zweimal je 10 ml Wasser gewaschen und eingeengt. Man erhält [trans-4-(1E-Pentenyl[trans-4-(1E-Pentenyl)cyclohexyl]malonaldehydtetramethylacetal als farbloses Oel.
c) Ein Gemisch aus 8,9 g [trans-4-(1E-Pentenyl)cyclohexyl]malonaldehydtetramethylacetal, 0,67 ml Wasser und 0,08 g p-Toluolsulfonsäure-monohydrat wird 1 Stunde zum Sieden erhitzt, abgekühlt, mit 0,22 g Natriumhydrogencarbonat versetzt und 15 Minuten nachgerührt. Die Suspension wird filtriert und der Rückstand mit Methanol gewaschen. Unterdessen wird eine Natriummethylat-Lösung aus 1,06 g Natrium und 45 ml Methanol bereitet. Diese Lösung wird zunächst mit 6,06 g p-Chlorbenzamidinhydroclorid und anschliessend innert 20 Minuten mit der obigen 3-Methoxy-2-[trans-4-(1E-pentenyl)cyclohexyl]acrolein-Lösung versetzt. Das Gemisch wird 18 Stunden gerührt, mit 2,5 ml 25-proz. Salzsäure bei pH 5 neutralisiert und auf -20°C gekühlt. Die Suspension wird genutscht, der Rückstand mit Methanol (-20°C) gewaschen, die Filtrate vereinigt, getrocknet und eingeengt. Der Eindampf-Rückstand wird in Diäthyläther aufgenommen, mit Wasser gewaschen, getrocknet und eingedampft. Der Rückstand ergibt nach Umkristallisation aus Aethylacetat analysenreines 2-(4-Chlorphenyl)-5-[trans-4-(1E-pentenyl)cyclohexyl]pyrimidin.

Auf analoge Weise können hergestellt werden:
2-(3,4-Difluorphenyl)-5-(trans-4-vinylcyclohexyl)pyrimidin, Smp. (C-N) 96,0°C, Klp. (N-I) 109,8°C
2-(4-Chlor-3-fluorphenyl)-5-(trans-4-vinylcyclohexyl)pyrimidin,
2-(3-Chlor-4-fluorphenyl)-5-(trans-4-vinylcyclohexyl)pyrimidin,
2-(4-Chlorphenyl)-5-(trans-4-vinylcyclohexyl)pyrimidin, Smp. (C-N) 129,7°C, Klp. (N-I) 178,5°C,
2- (3,4-Dichlorphenyl)-5-(trans-4-vinylcyclohexyl)pyrimidin,
2-(3,4-Difluorphenyl)-5-[trans-4-(1E-propenyl)cyclohexyl]-pyrimidin,
2-(4-Chlor-3-fluorphenyl)-5-[trans-4-(1E-propenyl)cyclohexyl]pyrimidin,
2-(3-Chlor-4-fluorphenyl)-5-[trans-4-(1E-propenyl)cyclohexyl]pyrimidin,
2-(4-Chlorphenyl)-5-[trans-4-(1E-propenyl)cyclohexyl]pyrimidin,
2-(3,4-Dichlorphenyl)-5-[trans-4-(1E-propenyl)cyclohexyl]-pyrimidin,
2-(3,4-Difluorphenyl)-5-[trans-4-(1E-butenyl)cyclohexyl]pyrimidin,
2-(4-Chlor-3-fluorphenyl)-5-[trans-4-(1E-butenyl)cyclohexyl]pyrimidin,
2-(3-Chlor-4-fluorphenyl)-5-[trans-4-(1E-butenyl)cyclohexyl]pyrimidin,
2-(4-Chlorphenyl)-5-[trans-4-(1E-butenyl)cyclohexyl]pyrimidin,
2-(3,4-Dichlorphenyl)-5-[trans-4-(1E-butenyl)cyclohexyl]pyrimidin,
2-(3,4-Difluorphenyl)-5-[trans-4-(1E-pentenyl)cyclohexyl]-pyrimidin,
2-(4-Chlor-3-fluorphenyl)-5-[trans+(1E-pentenyl)cyclohexyl]-pyrimidin,
2-(3-Chlor-4-fluorphenyl)-5-[trans-4-(1E-pentenyl)cyclohexyl]-pyrimidin,
2-(3,4-Dichlorphenyl)-5-[trans+(1E-pentenyl)cyclohexyl]-pyrimidin,
2-(3,4-Difluorphenyl)-5-{2-[trans-4-(vinyl)cyclohexyl]äthyl}pyrimidin,
2-(4-Chlor-3-fluorphenyl)-5-{2-[trans-4-(vinyl)cyclohexyl]äthyl}pyrimidin,
2-(3-Chlor-4-fluorphenyl)-5-{2-[trans-4-(vinyl)cyclohexyl]äthyl}pyrimidin,
2-(4-Chlorphenyl)-5-(2-[trans-4-(vinyl)cyclohexyl]äthyl)pyrimidin,Smp. (C-N) 109,9°C, Klp. (N-I) 144,4°C,
2-(3,4-Dichlorphenyl)-5-{2-[trans-4-(vinyl)cyclohexyl]äthyl}pyrimidin,
2-(3,4-Difluorphenyl)-5-{2-[trans+(1E-propenyl)cyclohexyl]äthyl}pyrimidin,
2-(4-Chlor-3-fluorphenyl)-5-{2-[trans-4-(1E-propenyl)cyclohexyl]äthyl}pyrimidin, pyrimidin,
2-(3-Chlor-4-fluorphenyl)-5-(2-[trans-4-E-propenyl)cyclohexyl]äthyl}pyrimidin,
2-(4-Chlorphenyl)-5-(2-[trans-4-(1E-propenyl)cyclohexyl]äthyl}pyrimidin,
2-(3,4-Dichlorphenyl)-5{2 [trans-4-(1E-propenyl)cyclohexyl]äthyl}pyrimidin,
2-(3,4-Difluorphenyl)-5-(2-[trans-4-(1E-butenyl)cyclohexyl]äthyl)pyrimidin,
2-(4-Chlor-3-fluorphenyl)-5-(2-[trans+(1E-butenyl)cyclohexyl]äthyl}pyrimidin,
2-(3-Chlor-4-fluorphenyl)-5-{2-[trans-4-(1E-butenyl)cyclohexyl]äthyl}pyrimidin,
2-(4-Chlorphenyl)-5-{2-[trans-4-(1E-butenyl)cyclohexyl]äthyl}pyrimidin,
2-(3,4-Dichlorphenyl)-5-{2-[trans-4-(1E-butenyl)cyclohexyl]äthyl}pyrimidin,
2-(3,4-Difluorphenyl)-5-{2-[trans-4-(1E-pentenyl)cyclohexyl]äthyl}pyrimidin,
2-(4-Chlor-3-fluorphenyl)-5-{2-[trans-4-(1E-pentenyl)cyclohexyl]äthyl}pyrimidin,
2-(3-Chlor-4-fluorphenyl)-5-{2-[trans+(1E-pentenyl)cyclohexyl]äthyl}pyrimidin,
2-(4-Chlorphenyl)-5-{2-[trans-4-(1E-pentenyl)cyclohexyl]äthyl}pyrimidin,
2-(3,4-Dichlorphenyl)-5-{2-[trans-4-(1E-pentenyl)cyclohexyl]äthyl}pyrimidin.

### Beispiel 8

Zur Untersuchung der Eigenschaften der Verbindungen der Formel I in Gemischen wurden binäre Mischungen mit 4-(trans-4-Pentylcyclohexyl)benzonitril hergestellt. Die Schwellenspannung und die Ansprechzeiten wurden in einer TN-Zelle (low bias tilt) mit 8 mm Plattenabstand gemessen; als Betriebsspannung wurde der 2,5-fache Wert der Schwellenspannung gewählt. Die entsprechenden Daten für reines 4-(trans-4-Pentylcyclohexyl)benzonitril betragen: Klp. (N-I) 54,6°C, V10 = 1,62 V, tₒₙ = 30 ms, toff = 42 ms, Δn = 0,120.

| Mischung A | |
|---|---|
| 90 Gew.-% | 4-(trans-4-Pentylcyclohexyl)benzonitril, |
| 10 Gew.-% | 4-[trans-4-Vinylcyclohexyl]-3',4'-difluorbiphenyl |

Klp. (N-I) 55,2°C, V₁₀ = 1,42 V, tₒₙ = 29 ms, t_{off} = 42 ms, Δn = 0,124.

| Mischung B | |
|---|---|
| 80 Gew.-% | 4-(trans-4-Pentylcyclohexyl)benzonitril, |
| 20 Gew.-% | 4-[trans-4-Vinylcyclohexyl]-3',4'-difluorbiphenyl |

Klp. (N-I) 55,8°C, V₁₀ = 1,35 V, tₒₙ = 33 ms, t_{off} = 46 ms, Δn = 0,126.

| Mischung C | |
|---|---|
| 90 Gew.-% | 4-(trans-4-Pentylcyclohexyl)benzonitril, |
| 10 Gew.-% | 4-[trans-4-(1E-Propenyl)cyclohexyl]-3',4'-difluorbiphenyl |

Klp. (N-I) 57,8°C, V₁₀ = 1,49 V, tₒₙ = 25 ms, t_{off} = 41 ms, Δn = 0,126.

| Mischung D | |
|---|---|
| 80 Gew.-% | 4-(trans-4-Pentylcyclohexyl)benzonitril, |
| 20 Gew.-% | 4-[trans-4-(1E-Propenyl)cydohexyl]-3',4'-difluorbiphenyl |

Klp. (N-I) 61,1°C, V₁₀ = 1,51 V, tₒₙ = 26 ms, t_{off} = 42 ms, Δn = 0,129.

| Mischung E | |
|---|---|
| 0 Gew.-% | 4-(trans-4-Pentylcyclohexyl)benzonitril |
| 10 Gew.-% | 4-[trans-4-(1E-Butenyl)cyclohexyl]-3',4'-difluorbiphenyl |

Klp. (N-I) %,6°C, V₁₀ = 1,56 V, tₒₙ = 25 ms, t_{off} = 44 ms, Δn = 0,125.

| Mischung F | |
|---|---|
| 80 Gew.-% | 4-(trans-4-Pentylcyclohexyl)benzonitril |
| 20 Gew.-% | 4-[trans-4-(1E-Butenyl)cyclohexyl]-3',4'-difluorbiphenyl |

Klp. (N-I) 58,9°C, V₁₀ = 1,61 V, tₒₙ = 26 ms, t_{off} = 45 ms, Δn = 0,127.

| Mischung G | |
|---|---|
| 90 Gew.-% | 4-(trans-4-Pentylcyclohexyl)benzonitril |
| 10 Gew.-% | 4-[trans-4-(1E-Pentenyl)cydohexyl]-3',4'-difluorbiphenyl |

Klp. (N-I) 56,8°C, V₁₀ = 1,62 V, tₒₙ = 25 ms, t_{off} = 42 ms, Δn = 0,122.

| Mischung H | |
|---|---|
| 80 Gew.-% | 4-(trans-4-Pentylcyclohexyl)benzonitril |
| 20 Gew.-% | 4-[trans-4-(1E-Pentenyl)cyclohexyl]-3',4'-difluorbiphenyl |

Klp. (N-I) 60,8°C, V₁₀ = 1,60 V, tₒₙ = 29 ms, t_{off} = 49 ms, Δn = 0,124.

| Mischung I | |
|---|---|
| 90 Gew.-% | 4-(trans-4-Pentylcyclohexyl)benzonitril |
| 10 Gew.-% | 4-(trans-4-Vinylcyclohexyl)-4'-chlor-3'-fluorphenyl |

Klp. (N-I) 58,1°C, V₁₀ = 1,65 V, tₒₙ = 25 ms, t_{off} = 43 ms, Δn = 0,130.

| Mischung J | |
|---|---|
| 80 Gew.-% | 4-(trans-4-Pentylcyclohexyl)benzonitril |
| 20 Gew.-% | 4-(trans-4-Vinylcyclohexyl)-4'-chlor-3'-fluorphenyl |

Klp. (N-I) 62,1°C, V₁₀ = 1,66 V, tₒₙ = 26 ms, t_{off} = 42 ms, Δn = 0,134.

| Mischung K | |
|---|---|
| 90 Gew.-% | 4-(trans-4-Pentylcyclohexyl)benzonitril |
| 10 Gew.-% | 4-[trans-4-(1E-Propenyl)cyclohexyl]-4'-chlor-3'-fluorbiphenyl |

Klp. (N-I) 60,6°C, V₁₀ = 1,68 V, tₒₙ = 25 ms, t_{off} = 43 ms, Δn = 0,131.

| Mischung L | |
|---|---|
| 80 Gew.-% | 4-(trans-4-Pentylcyclohexyl)benzonitril |
| 20 Gew.-% | 4-[trans-4-(1E-Propenyl)cyclohexyl]-4'-chlor-3'-fluorbiphenyl |

Klp. (N-I) 68,7°C, V₁₀ = 1,78 V, tₒₙ = 26 ms, t_{off} = 45 ms, Δn = 0,137.

| Mischung M | |
|---|---|
| 90 Gew.-% | 4-(trans-4-Pentylcyclohexyl)benzonitril |
| 10 Gew.-% | 4-[trans-4-(1E-Butenyl)cyclohexyl]-4'-chlor-3'-fluorbiphenyl |

Klp. (N-I) 59,4°C, V₁₀ = 1,63 V, tₒₙ = 26 ms, t_{off} = 43 ms, Δn = 0,129.

| Mischung N | |
|---|---|
| 80 Gew.-% | 4-(trans-4-Pentylcyclohexyl)benzonitril |
| 20 Gew.-% | 4-[trans-4-(1E-Butenyl)cyclohexyl]-4'-chlor-3'-fluorbiphenyl |

Klp. (N-I) 63,7°C, V₁₀ = 1,66 V, tₒₙ = 27 ms, t_{off} = 44 ms, Δn = 0,138.

| Mischung O | |
|---|---|
| 90 Gew.-% | 4-(trans-4-Pentylcyclohexyl)benzonitril |
| 10 Gew.-% | 4-[trans-4-(1E-Pentenyl)cyclohexyl]-4'-chlor-3'-fluorbiphenyl |

Klp. (N-I) 59,8°C, V₁₀ = 1,65 V, tₒₙ = 25 ms, t_{off} = 42 ms, Δn = 0,127.

| Mischung P | |
|---|---|
| 80 Gew.-% | 4-(trans-4-Pentylcyclohexyl)benzonltril |
| 20 Gew.-% | 4-[trans-4-(1E-Pentenyl)cyclohexyl]-4'-chlor-3'-fluorbiphenyl |

Klp. (N-I) 63,4°C, V₁₀ = 1,72 V, tₒₙ = 25 ms, t_{off} = 43 ms, Δn = 0,134.

| Mischung Q | |
|---|---|
| 90 Gew.-% | 4-(trans-4-Pentylcyclohexyl)benzonitril |
| 10 Gew.-% | 5-[2-(trans-4-Vinylcyclohexyl)äthyl]-2-[3,4-difluorphenyl)pyridin |

Klp. (N-I) 52,3°C, V₁₀ = 1,48 V, tₒₙ = 30 ms, t_{off} = 45 ms, Δn = 0,124.

| Mischung R | |
|---|---|
| 90 Gew.-% | 4-(trans-4-Pentylcyclohexyl)benzonitril |
| 10 Gew.-% | 5-[2-[trans-4-(1E-Propenyl)cyclohexyl]äthyl]-2-(3,4-difluorphenyl)pyridin |

Klp. (N-I) 57,1°C, V₁₀ = 1,63 V, tₒₙ = 26 ms, t_{off} = 44 ms, Δn = 0,125.

| Mischung S | |
|---|---|
| 80 Gew.-% | 4-(trans-4-Pentylcyclohexyl)benzonitril |
| 20 Gew.-% | 5-[2-[trans-4-(1E-Propenyl)cyclohexyl]äthyl]-2-(3,4-difluorphenyl)pyridin |

Klp. (N-I) 60,2°C, V₁₀ = 1,59 V, tₒₙ = 30 ms, t_{off} = 49 ms, Δn = 0,130.

## Patentansprüche

1. Flüssigkristallines Gemisch mit mindestens 2 Komponenten, dadurch gekennzeichnet, daß mindestens eine Komponente eine Verbindung der Formel I 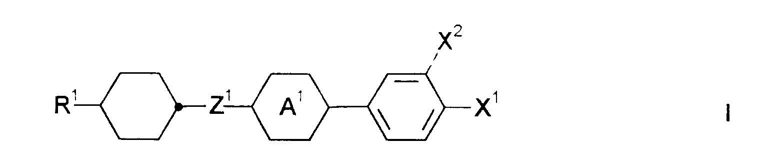 ist,
worin
Z¹ eine einfache Kovalenzbindung oder -CH₂CH₂-bezeichnet;
Ring A¹ 1,4-Phenylen, Pyrimidin-2,5-diyl, Pyridin-2,5-diyl oder, wenn Z¹ für -CH₂CH₂- steht, auch trans-1,4-Cyclohexylen darstellt;
X¹ für Fluor oder Chlor steht;
X² Fluor oder Chlor bedeutet,
R¹ Vinyl oder 1E-Alkenyl mit 3 bis 12 Kohlenstoffatomen bedeutet,
wobei der Anteil an Verbindungen der Formel I etwa 5-30 Gew.-% beträgt.

2. Flüssigkristallines Gemisch nach Anspruch 1, dadurch gekennzeichnet, daß es Verbindungen der allgemeinen Formeln 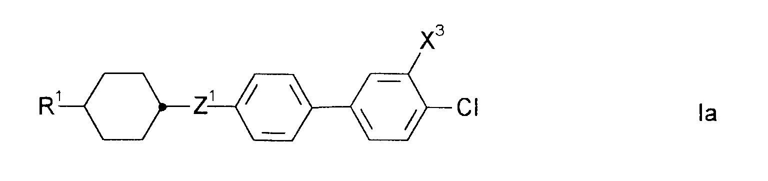 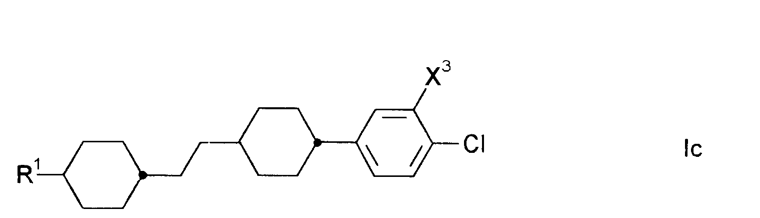 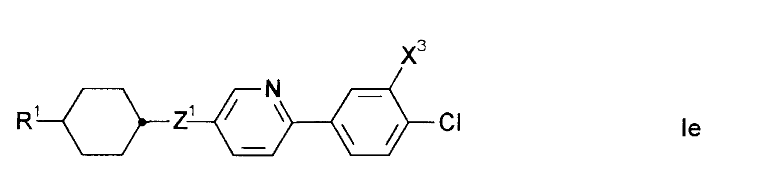 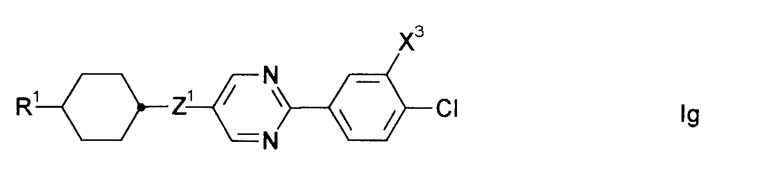 enthält,
worin
R¹ Vinyl oder einen 1E-Alkenylrest mit 3 bis 12 Kohlenstoffatomen darstellt;
Z¹ eine einfache Kovalenzbindung oder -CH₂CH₂- bedeutet;
X³ Chlor oder Fluor bezeichnet.

3. Flüssigkristallines Gemisch nach Anspruch 2, dadurch gekennzeichnet, daß Z¹ eine einfache Kovalenzbindung bedeutet.

4. Flüssigkristallines Gemisch nach Anspruch 1, gekennzeichnet durch die allgemeinen Formeln 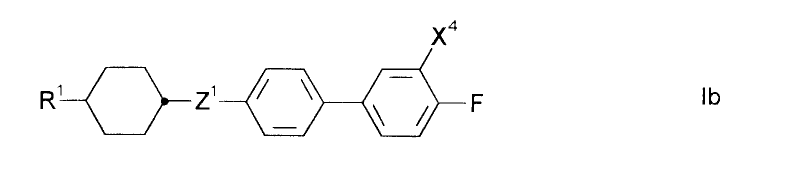 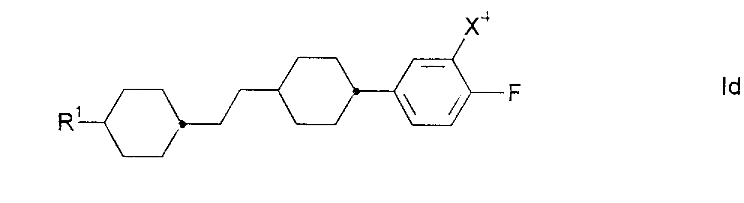 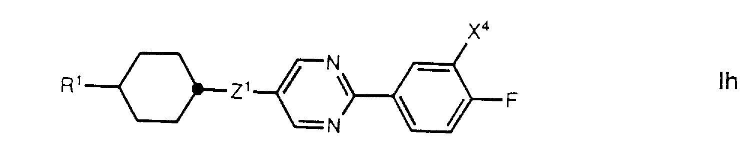 worin
R¹ Vinyl oder einen 1E-Alkenylrest mit 3 bis 12 Kohlenstoffatomen darstellt;
Z¹ eine einfache Kovalenzbindung oder -CH₂CH₂- bedeutet:
X⁴ Chlor oder Fluor bezeichnet.

5. Flüssigkristallines Gemisch nach Anspruch 1, gekennzeichnet durch die allgemeine Formel 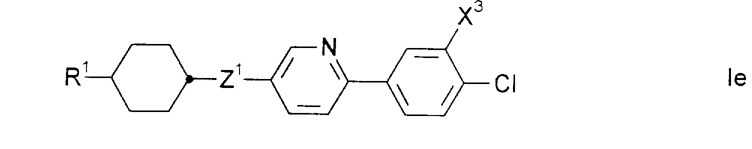 worin
R¹ Vinyl oder einen 1E-Alkenylrest mit 3 bis 12 Kohlenstoffatomen darstellt;
Z¹ -CH₂CH₂- bedeutet:
X³ Chlor oder Fluor bezeichnet.

6. Fiüssigkristallines Gemisch nach einem der Ansprüche 1 bis 5, dadurch gekennzeichnet, daß R¹ einen geradkettigen Rest bedeutet.

7. Flüssigkristallines Gemisch nach einem der Ansprüche 1 bis 6, dadurch gekennzeichnet, daß R¹ 2 bis 7, vorzugsweise 2 bis 5 Kohlenstoffatome, aufweist.

8. Flüssigkristallines Gemisch nach einem der Ansprüche 1 bis 7, dadurch gekennzeichnet, daß es eine oder mehrere Verbindungen der Formel I und eine oder mehrere Verbindungen aus der Gruppe der Verbindungen der allgemeinen Formeln 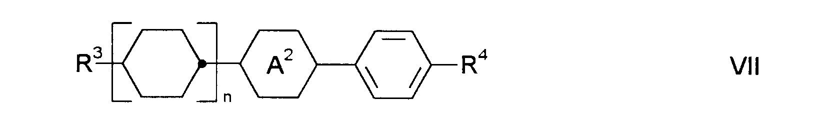 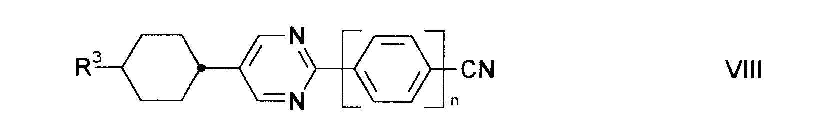 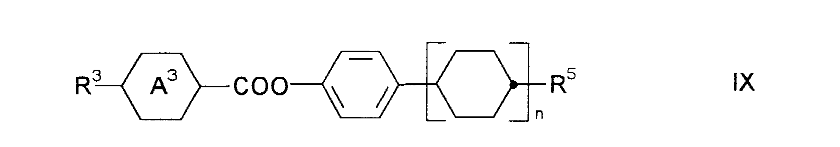 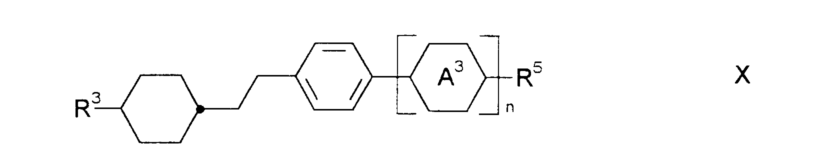 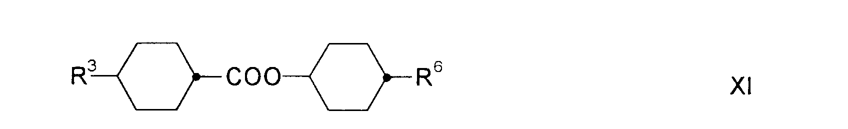 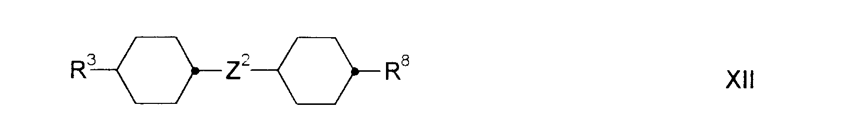 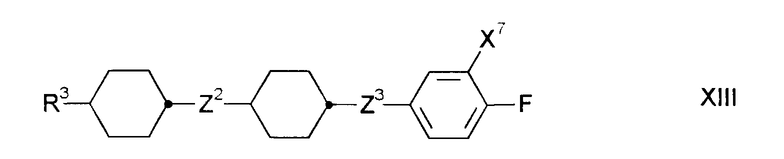 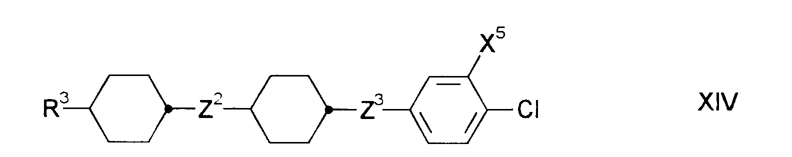 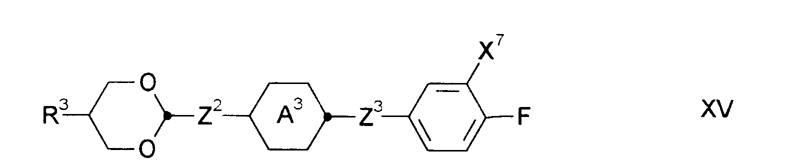 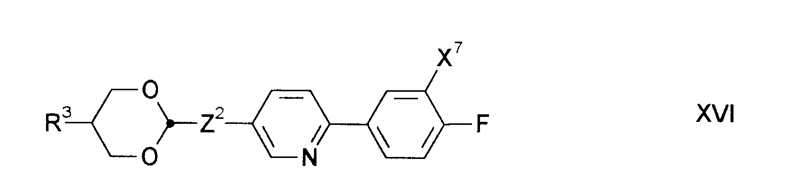 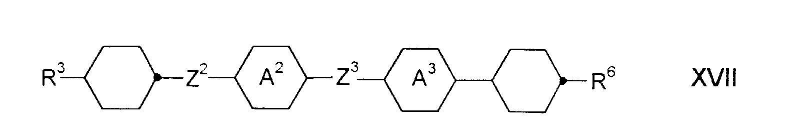 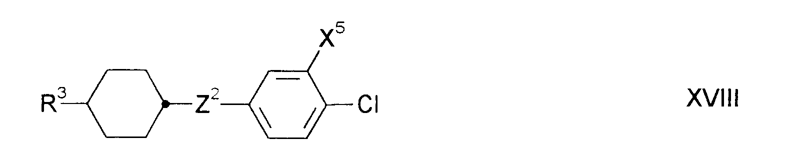 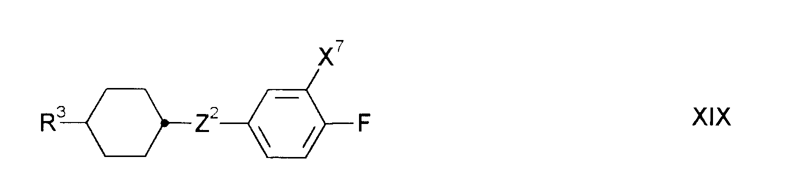 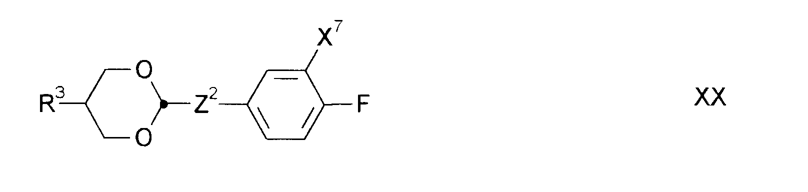 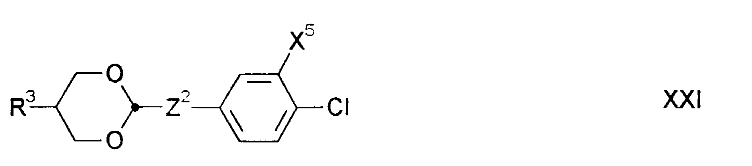 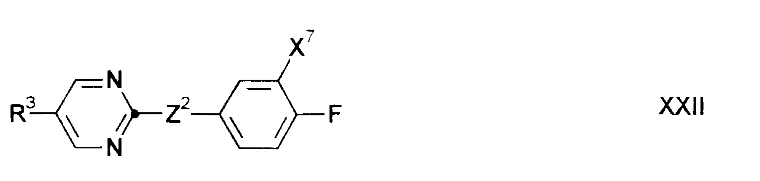 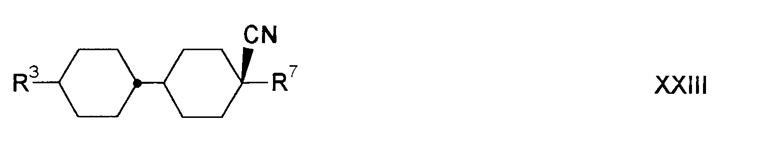 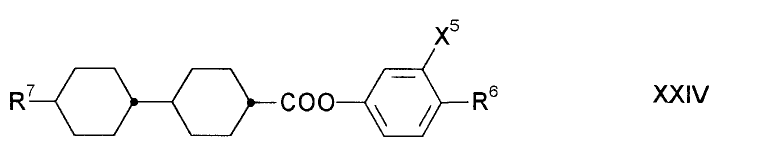 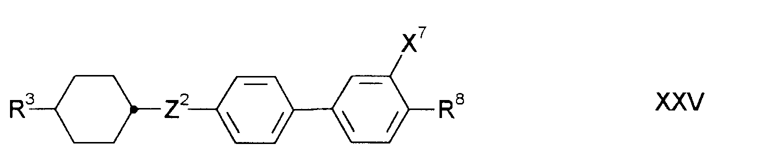 worin n für die Zahl 0 oder 1 steht; R³ und R⁶ unabhängig voneinander Alkyl, 3E-Alkenyl,4-Alkenyl, Alkoxyalkyl oder an einem gesättigten Ring auch 1E-Alkenyl bedeutet; Ring A² 1,4-Phenylen, trans-1,4-Cyclohexylen, Pyridin-2,5-diyl, Pyrimidin-2,5-diyl oder trans-1,3-Dioxan-2,5-diyl darstellt; R⁴ Cyano, -NCS, Fluor, Alkyl, 3E-Alkenyl, 4-Alkenyl, Alkoxy, 2E-Alkenyloxy, 3-Alkenyloxy oder 1-Alkinyl bezeichnet; Ring A³ 1,4-Phenylen oder trans-1,4-Cyclohexylen darstellt; R⁵ Alkyl, 3E-Alkenyl, 4-Alkenyl oder an einem Cyclohexanring auch 1E-Alkenyl oder an einem Benzolring auch Cyano, -NCS, Alkoxy, 2E-Alkenyloxy oder 3-Alkenyloxy bedeutet; R⁷ Alkyl, 1E-Alkenyl, 3E-Alkenyl oder 4-Alkenyl bezeichnet; Z² und Z³ unabhängig voneinander eine einfache Kovalenzbindung oder -CH₂CH₂- darstellt, wobei zwei aromatische Ringe immer durch eine einfache Kovalenzbindung verknüpft sind; R⁸ Cyano, Alkyl, 3E-Alkenyl, 4-Alkenyl, Alkoxy, 2E-Alkenyloxy, 3-Alkenyloxy, Alkoxymethyl, (2E-Alkenyl)oxymethyl oder an einen Cyclohexanring auch 1E-Alkenyl bedeutet; X⁵ Wasserstoff, Chlor oder Fluor bezeichnet; X⁶ Cyano, Chlor oder Fluor bedeutet; und X⁷ Wasserstoff oder Fluor bedeutet,
enthält.

9. Verwendung des flüssigkristallinen Gemisches nach Anspruch 1 für elektro-optische Zwecke.

## Claims

1. Liquid-crystalline mixture having at least 2 components, characterized in that at least one component is a compound of the formula I 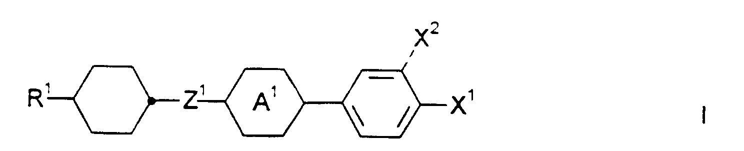 in which
Z¹ is a single covalent bond or -CH₂CH₂-;
ring A¹ is 1,4-phenylene, pyrimidine-2,5-diyl, pyridine-2,5-diyl or, if Z¹ is -CH₂CH₂-, alternatively trans-1,4-cyclohexylene;
X¹ is fluorine or chlorine;
X² is fluorine or chlorine,
R¹ is vinyl or 1E-alkenyl having 3 to 12 carbon atoms,
where the proportion of compounds of the formula I is about 5-30% by weight.

2. Liquid-crystalline mixture according to Claim 1, characterized in that it comprises compounds of the general formula 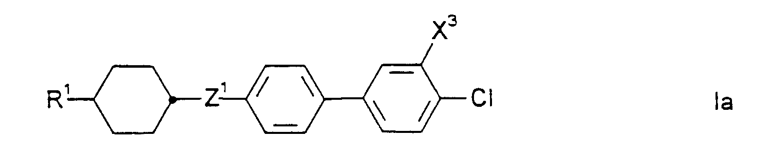 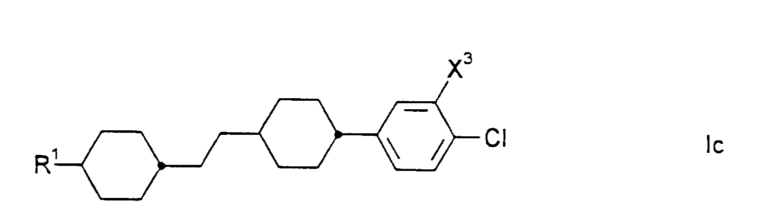 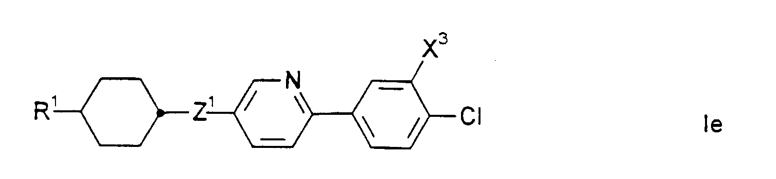 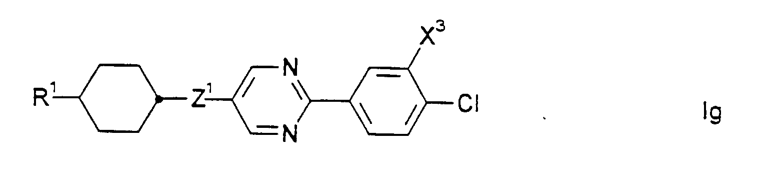 in which
R¹ is vinyl or a lE-alkenyl radical having 3 to 12 carbon atoms;
Z¹ is a single covalent bond or -CH₂CH₂-;
X³ is chlorine or fluorine.

3. Liquid-crystalline mixture according to Claim 2, characterized in that Z¹ is a single covalent bond.

4. Liquid-crystalline mixture according to Claim 1, characterized by the general formulae 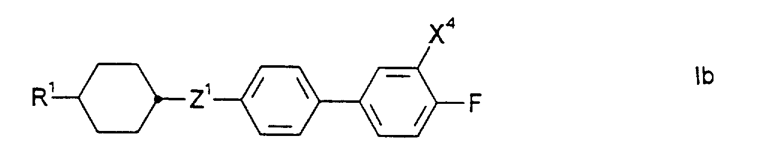 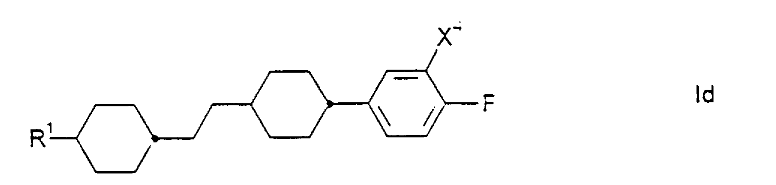 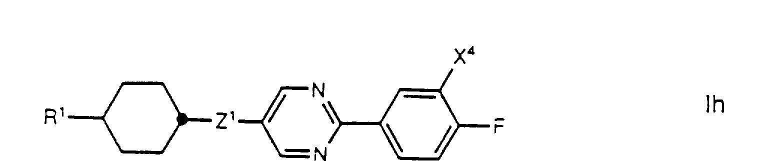 in which
R¹ is vinyl or a lE-alkenyl radical having 3 to 12 carbon atoms;
Z¹ is a single covalent bond or -CH₂CH₂-;
X⁴ is chlorine or fluorine.

5. Liquid-crystalline mixture according to Claim 1, characterized by the general formula 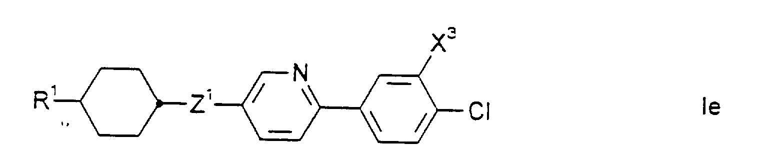 in which
R¹ is vinyl or a lE-alkenyl radical having 3 to 12 carbon atoms;
Z¹ is -CH₂CH₂-;
X³ is chlorine or fluorine.

6. Liquid-crystalline mixture according to one of Claims 1 to 5, characterized in that R¹ is a straight-chain radical.

7. Liquid-crystalline mixture according to one of Claims 1 to 6, characterized in that R¹ has from 2 to 7, preferably from 2 to 5, carbon atoms.

8. Liquid-crystalline mixture according to one of Claims 1 to 7, characterized in that it comprises one or more compounds of the formula I and one or more compounds from the group consisting of the compounds of the general formulae 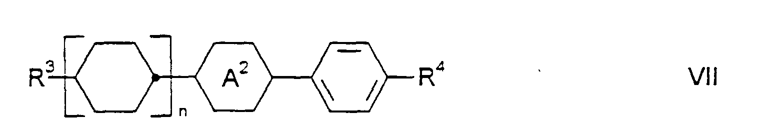 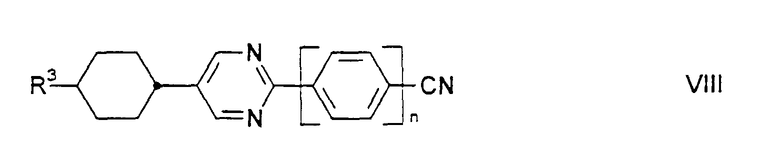 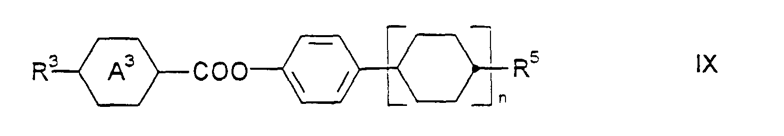 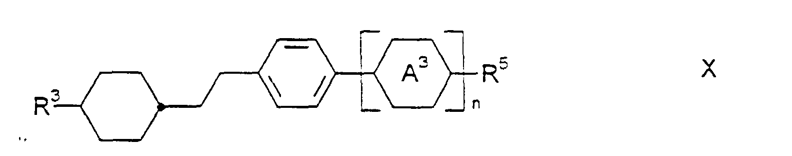 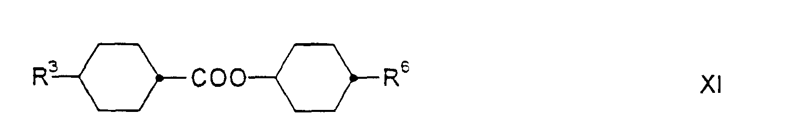 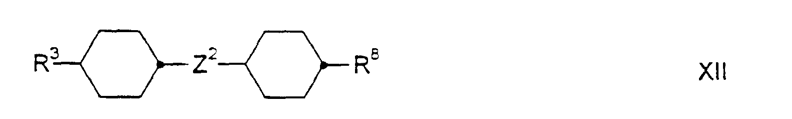 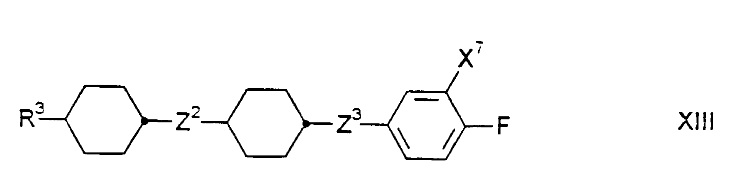 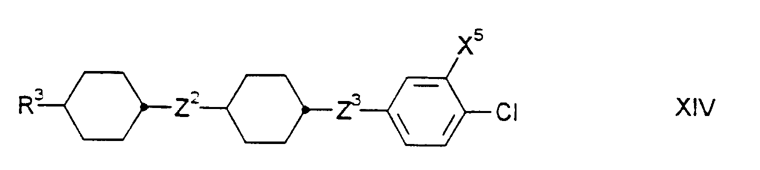 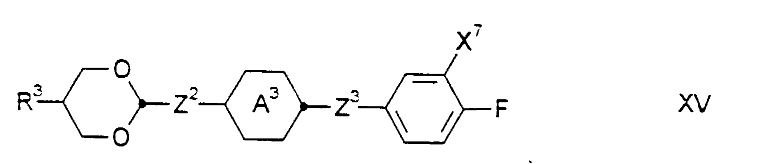 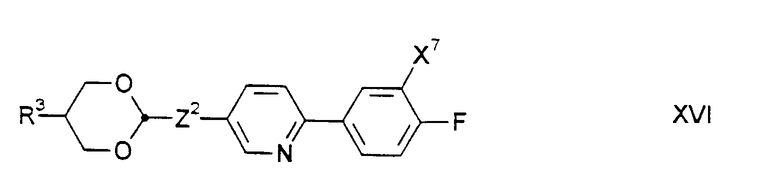 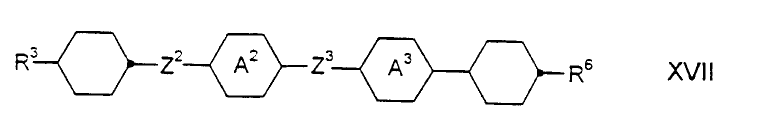 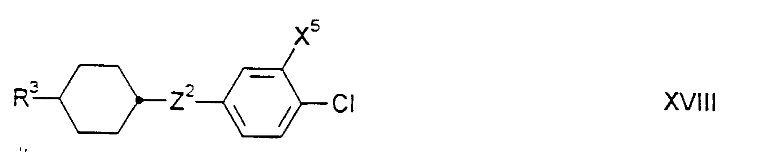 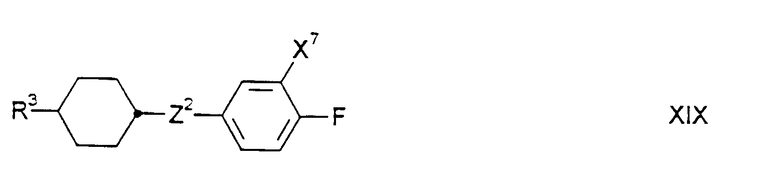 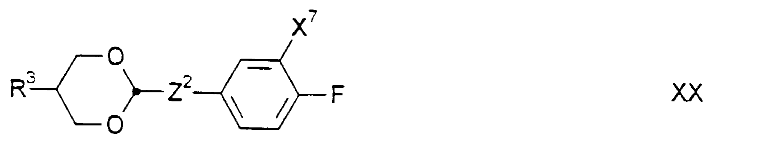 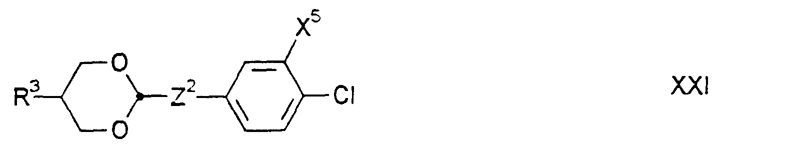 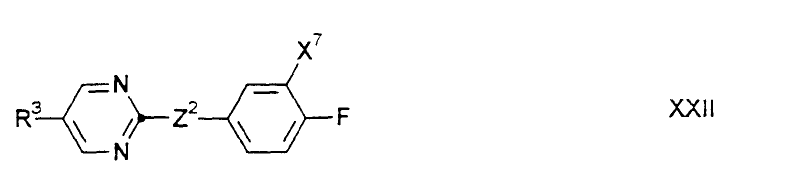 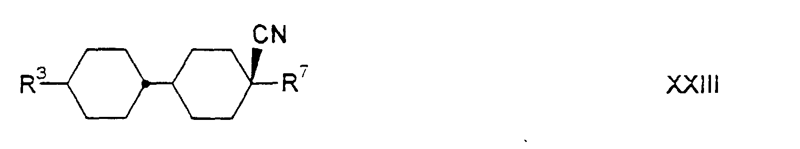 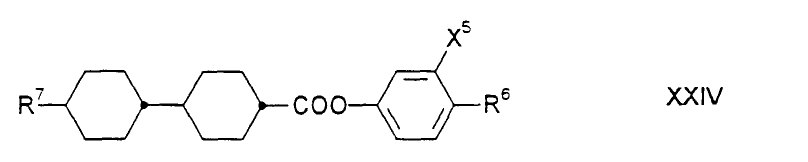 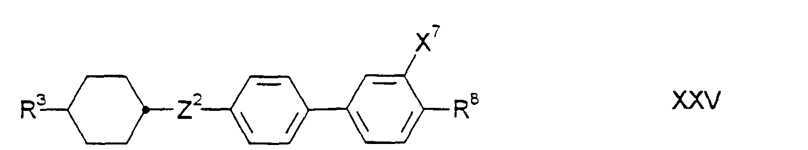 in which n is the number 0 or 1; R³ and R⁶, independently of one another, are alkyl, 3E-alkenyl, 4-alkenyl, alkoxyalkyl or, on a saturated ring, alternatively lE-alkenyl; ring A² is 1,4-phenylene, trans-1,4-cyclohexylene, pyridine-2,5-diyl, pyrimidine-2,5-diyl or trans-1,3 -dioxane-2,5-diyl; R⁴ is cyano, -NCS, fluorine, alkyl, 3E-alkenyl, 4-alkenyl, alkoxy, 2E-alkenyloxy, 3-alkenyloxy or 1-alkynyl; ring A³ is 1,4-phenylene or trans-1,4-cyclohexylene; R⁵ is alkyl, 3E-alkenyl, 4-alkenyl or, on a cyclohexane ring, alternatively 1E-alkenyl or, on a benzene ring, alternatively cyano, -NCS, alkoxy, 2E-alkenyloxy or 3-alkenyloxy; R⁷ is alkyl, lE-alkenyl, 3E-alkenyl or 4-alkenyl; Z² and Z³, independently of one another, are a single covalent bond or -CH₂CH₂-, where two aromatic rings are always linked by a single covalent bond; R⁸ is cyano, alkyl, 3E-alkenyl, 4-alkenyl, alkoxy, 2E-alkenyloxy, 3-alkenyloxy, alkoxymethyl, (2E-alkenyl)oxymethyl or, on a cyclohexane ring, alternatively 1E-alkenyl; x⁵ is hydrogen, chlorine or fluorine; X⁶ is cyano, chlorine or fluorine; and X⁷ is hydrogen or fluorine.

9. Use of the liquid-crystalline mixture according to Claim 1 for electro-optical purposes.

## Revendications

1. Composition de cristaux liquides à au moins deux composants, caractérisée en ce qu'au moins un composant est un composé de formule I 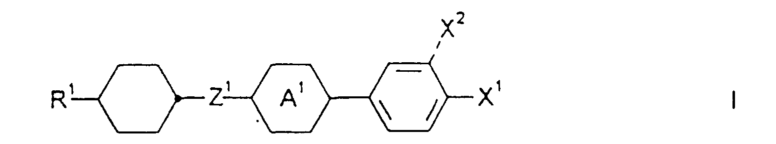 dans laquelle
Z¹ représente un liaison covalente simple ou CH₂CH₂-;
le cycle A¹ représente le groupe 1,4-phénylène, pyrimidine-2,5-diyle, pyridine-2,5-diyle ou, lorsque Z¹ représente -CH₂CH₂-, également le groupe *trans*-1,4-cyclohexylène;
X¹ représente un atome de fluor ou de chlore;
X² représente un atome de fluor ou de chlore;
R¹ représente le groupe vinyle ou un groupe lE-alcényle ayant de 3 à 12 atomes de carbone;
la proportions des composés de formule I allant environ de 5 à 30 % en poids.

2. Composition de cristaux liquides selon la revendication 1, caractérisée en ce qu'elle contient des composés de formules générales 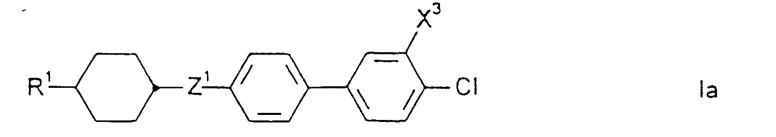 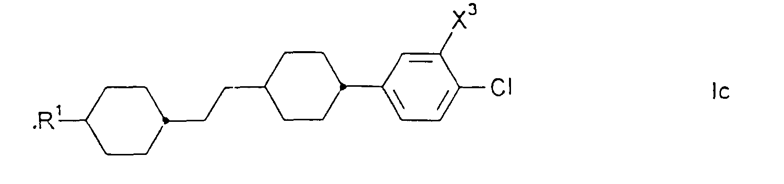 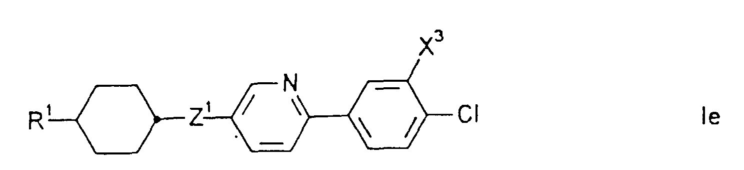 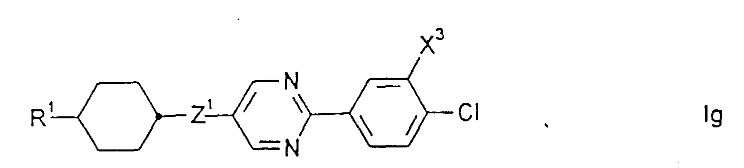 dans lesquelles
R¹ représente le groupe vinyle ou un radical lE-alcényle ayant de 3 à 12 atomes de carbone;
Z¹ représente un liaison covalente simple ou -CH₂CH₂-;
X³ représente un atome de chlore ou de fluor.

3. Composition de cristaux liquides selon la revendication 2, caractérisée en ce que Z¹ représente une liaison covalente simple.

4. Composition de cristaux liquides selon la revendication 1, caractérisée par les formules générales 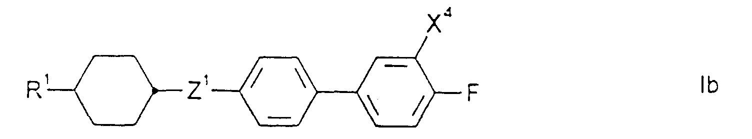 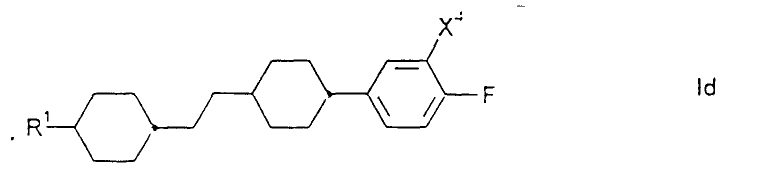 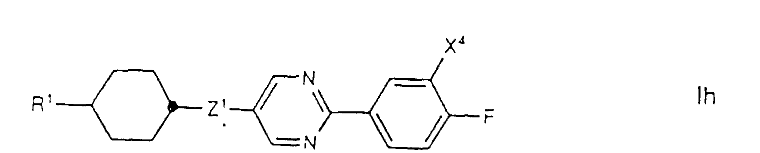 dans lesquelles
R¹ représente le groupe vinyle ou un radical lE-alcényle ayant de 3 à 12 atomes de carbone;
Z¹ représente une liaison covalente simple ou -CH₂CH₂-;
X⁴ représente un atome de chlore ou de fluor.

5. Composition de cristaux liquides selon la revendication 1, caractérisée par la formule générale 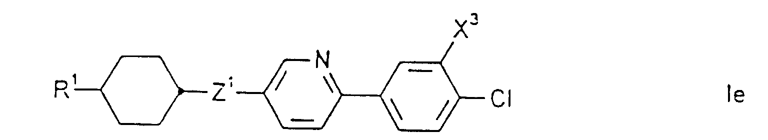 dans laquelle
R¹ représente le groupe vinyle ou un radical lE-alcényle ayant de 3 à 12 atomes de carbone;
Z¹ représente -CH₂CH₂-;
X³ représente un atome de chlore ou de fluor.

6. Composition de cristaux liquides selon l'une des revendications 1 à 5, caractérisée en ce que R¹ représente un radical à chaîne droite.

7. Composition de cristaux liquides selon l'une des revendications 1 à 6, caractérisée en ce que R¹ comporte de 2 à 7, de préférence de 2 à 5 atomes de carbone.

8. Composition de cristaux liquides selon l'une des revendications 1 à 7, caractérisée en ce qu'elle contient un ou plusieurs composés de formule I et un ou plusieurs composés choisis dans l'ensemble des composés de formules générales 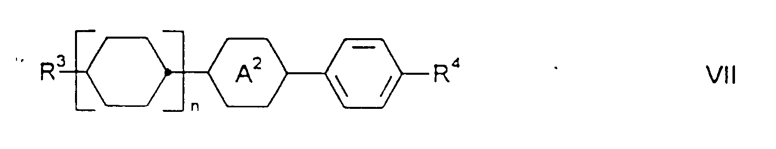 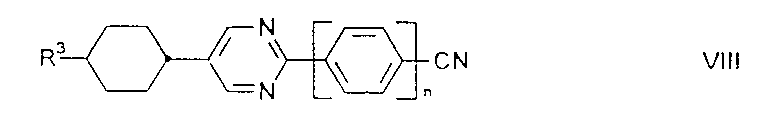 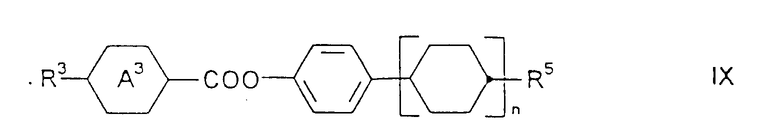 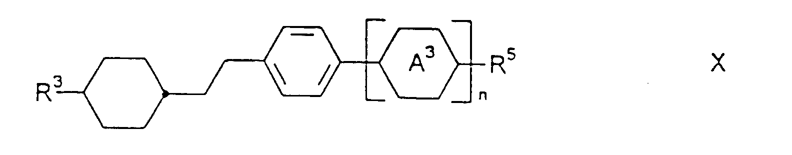 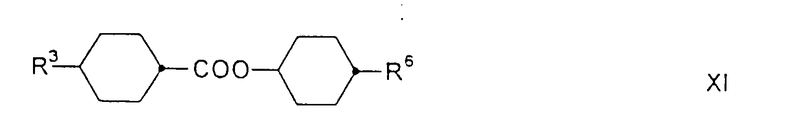 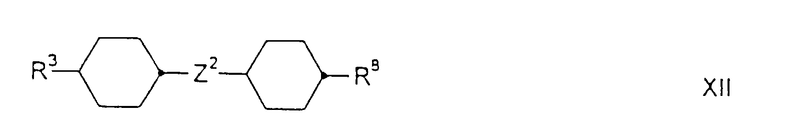 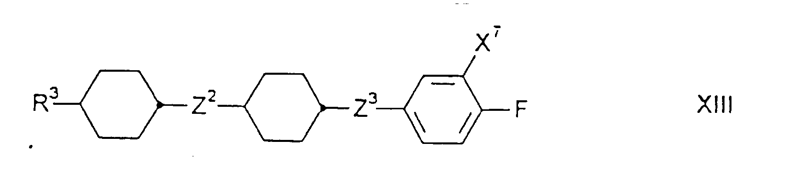 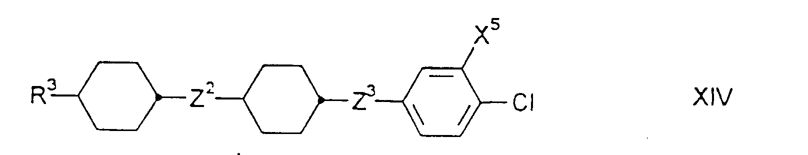 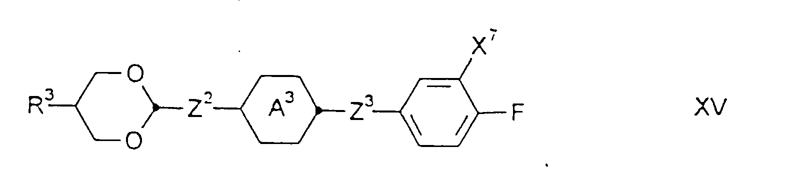 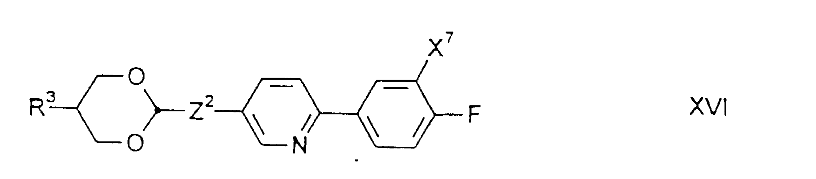 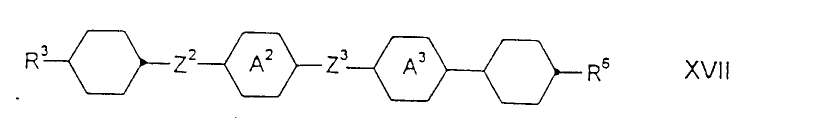 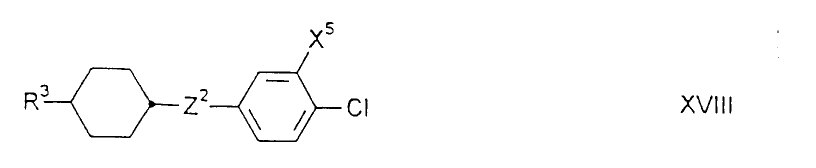 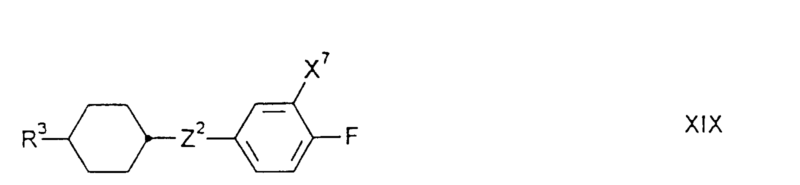 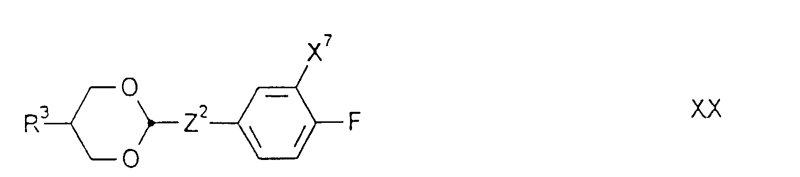 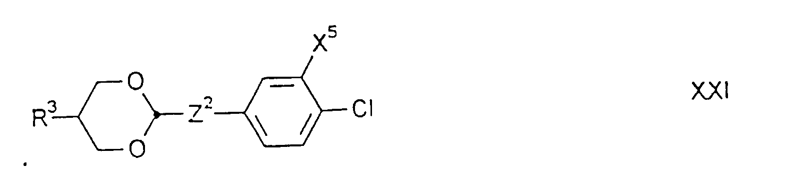 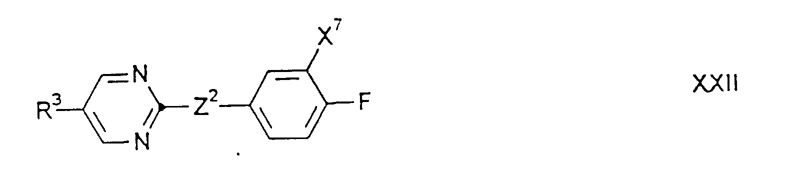 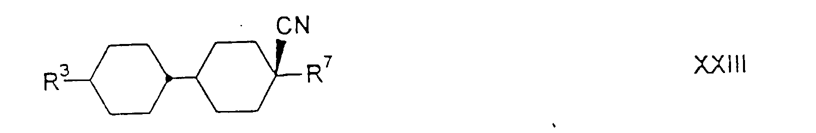 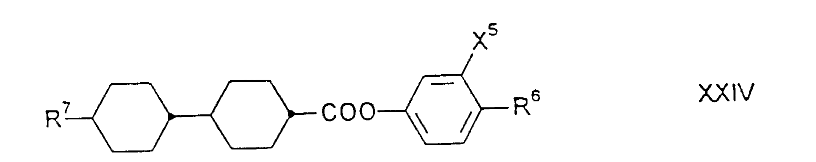 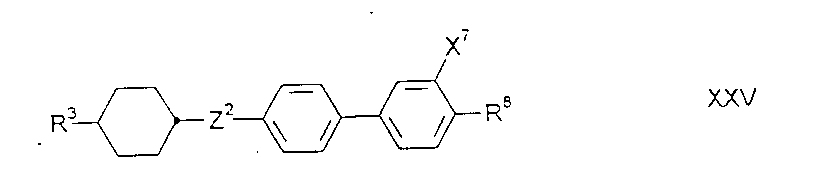 dans lesquelles n représente le nombre 0 ou 1; R³ et R⁶ représentent, indépendamment l'un de l'autre, un groupe alkyle, 3E-alcényle, 4-alcényle, alcoxyalkyle ou, sur un cycle saturé, également un groupe lE-alcényle; le cycle A² représente le groupe 1,4-phénylène, trans-1,4-cyclohexylène, pyridine-2,5-diyle, pyrimidine-2,5-diyle ou *trans*-1,3-dioxanne-2,5-diyle; R⁴ représente le groupe cyano, -NCS, un atome de fluor, un groupe alkyle, 3E-alcényle, 4-alcényle, alcoxy, 2E-alcényloxy, 3E-alcényloxy ou 1-alcynyle; le cycle A³ représente le groupe 1,4-phénylène ou trans-1,4-cyclohexylène; R⁵ représente un groupe alkyle, 3E-alcényle, 4E-alcényle ou, sur un cycle cyclohexane, également un groupe lE-alcényle, ou, sur un noyau benzénique, également un groupe cyano, -NCS, alcoxy, 2E-alcényloxy ou 3-alcényloxy; R⁷ représente un groupe alkyle, 1E-alcényle, 3E-alcényle ou 4-alcényle; Z² et Z³ représentent, indépendamment l'un de l'autre, une liaison covalente simple ou -CH₂CH₂-, deux noyaux aromatiques étant toujours reliés par une liaison covalente simple; R⁸ représente un groupe cyano, alkyle, 3E-alcényle, 4-alcényle, alcoxy, 2E-alcényloxy, 3E-alcényloxy, alcoxyméthyle, (2E-alcényl)oxyméthyle ou, sur un cycle cyclohexane, également un groupe lE-alcényle; X⁵ représente un atome d'hydrogène, de chlore ou de fluor; X⁶ représente le groupe cyano ou un atome de chlore ou de fluor; et X⁷ représente un atome d'hydrogène ou de fluor.

9. Utilisation de la composition de cristaux liquides selon la revendication 1, à des fins électro-optiques.
